# EUROPÄISCHE PATENTSCHRIFT

(11) **EP 4 035 133 B1**
(45) Veröffentlichungstag und Bekanntmachung des Hinweises auf die Patenterteilung: **21.05.2025**
(21) Anmeldenummer: 20792534.8
(22) Anmeldetag: 24.09.2020
(51) Int. Cl.: G07D 11/14, G07D 11/16, G07D 11/22

(54) **VORRICHTUNG ZUR HANDHABUNG VON WERTSCHEINEN**
APPARATUS FOR HANDLING NOTES OF VALUE
DISPOSITIF DE MANIPULATION DE DOCUMENTS DE VALEUR

(30) Priorität: 26.09.2019 DE 102019125994
(43) Veröffentlichungstag der Anmeldung: 03.08.2022
(73) Patentinhaber: Diebold Nixdorf Systems GmbH, 33106 Paderborn (DE)
(72) Erfinder: DÜSTERHUS, Richard, 33106 Paderborn (DE); SCHNIEDERMANN, Gerhard, 33098 Paderborn (DE)
(86) Internationale Anmeldenummer: PCT/EP2020/076657
(87) Internationale Veröffentlichungsnummer: WO 2021/058619

(56) Entgegenhaltungen:
- EP-A1- 3 115 973
- WO-A1-2014/155645
- WO-A1-2018/013622

## Beschreibung

Die Erfindung betrifft eine Vorrichtung zur Handhabung von Wertscheinen, die ein Ausgabefach zur Entnahme von Wertscheinen umfasst. Das Ausgabefach wird auf einer ersten Seite von einem Ablageelement und auf einer zweiten Seite von einem Begrenzungselement begrenzt. Ferner umfasst die Vorrichtung eine Steuereinheit.

Aus dem Stand der Technik sind Vorrichtungen zur Handhabung von Wertscheinen, insbesondere Geldautomaten, bekannt, bei denen die Wertscheine einem Benutzer als Bündel angeboten werden. Dazu werden die Wertscheine aus Wertscheinkassetten entnommen, einem Zwischenspeicher zugeführt und von einer Stapeleinheit zu einem Wertscheinstapel gestapelt, der dann mit Hilfe einer Transporteinheit, beispielsweise mit Hilfe eines Verfahrschlittens, zum Ausgabefach transportiert wird.

Nachteilig an diesen Vorrichtungen ist es, dass für den Zwischenspeicher, die Stapeleinheit und die Transporteinheit viel Bauraum benötigt wird und dass insbesondere die Zwischenspeicherung den Auszahlungsvorgang verlangsamt.

EP 3 115 973 A1 offenbart eine Vorrichtung zum Handhaben von Wertscheinen mit einem Ausgabefach. Das Ausgabefach weist ein verfahrbares Ablageelement auf, welches von einer Initialisierungsposition zu einer Zuführposition verfahren werden kann.

Es ist Aufgabe der Erfindung, eine Vorrichtung zur Handhabung von Wertscheinen anzugeben, mit deren Hilfe auf einfache Weise Wertscheine zur Ausgabe bereitgestellt werden können.

Diese Aufgabe wird durch eine Vorrichtung mit den Merkmalen des Anspruchs 1 gelöst. Vorteilhafte Weiterbildungen sind in den abhängigen Ansprüchen angegeben.

Die Vorrichtung mit den Merkmalen des Anspruchs 1 umfasst mindestens eine erste Sensoreinheit zum Erfassen einer Initialisierungsposition des Ablageelements. Die erste Sensoreinheit ist dazu ausgebildet, ein erstes Sensorsignal zu erzeugen und an die Steuereinheit zu übermitteln. Die Steuereinheit ist dazu ausgebildet, eine erste Antriebseinheit zum Bewegen des Ablageelements anzusteuern, wobei die Steuereinheit ausgehend von dem ersten Sensorsignal die erste Antriebseinheit derart ansteuert, dass das Ablageelement um eine vorbestimmte Strecke von dem Begrenzungselement weg von der Initialisierungsposition in eine erste Zuführposition zum Zuführen der Wertscheine bewegt wird. Dadurch wird eine besonders einfache und zuverlässige Steuerung der Bewegung des Ablageelements in die Zuführposition erreicht.

Besonders vorteilhaft ist es, wenn die die erste Sensoreinheit mindestens ein Sensorelement umfasst, wobei die erste Sensoreinheit derart angeordnet ist, dass mit Hilfe der ersten Sensoreinheit eine erste Position des Sensorelements detektierbar ist, und dass die erste Sensoreinheit dazu ausgebildet ist, das erste Sensorsignal in Abhängigkeit von der detektieren ersten Position des Sensorelements auszugeben. Dadurch wird eine besonders zuverlässige Überwachung der Position des Sensorelements erreicht.

Es ist besonders vorteilhaft, wenn die erste Sensoreinheit die erste Position des Sensorelements detektiert, wenn sich das Ablageelement in der Initialisierungsposition befindet, wobei das Sensorelement das Ablageelement kontaktiert und durch den Kontakt mit dem Ablageelement in Richtung des Begrenzungselements bewegt wird. Dadurch wird eine einfache und genaue Ermittlung der Initialisierungsposition erreicht.

Bei einer besonders vorteilhaften Ausführungsform ist das Sensorelement ein Andruckelement zur geordneten Ablage der Wertscheine, das schwenkbar mit dem Begrenzungselement verbunden ist, wobei das Sensorelement zwischen der angehobenen ersten Position und einer abgesenkten zweiten Position bewegbar ist. Dadurch wird eine unkontrollierte Bewegung der Wertscheine beim Zuführen in das Ausgabefach verhindert. Dadurch wird ein geordneter Wertscheinstapel im Ausgabefach gebildet.

Es ist vorteilhaft, wenn die Vorrichtung mindestens eine zweite Sensoreinheit zum Erfassen einer unteren Position des Ablageelements umfasst, wobei die zweite Sensoreinheit dazu ausgebildet ist, ein zweites Sensorsignal zu erzeugen und an die Steuereinheit zu übermitteln. Ferner ist die Steuereinheit dazu ausgebildet, die erste Antriebseinheit zum Bewegen des Ablageelements in Abhängigkeit des zweiten Sensorsignals anzusteuern. Dadurch wird eine einfache und zuverlässige Steuerung der Bewegung des Ablageelements erreicht.

Ferner ist es vorteilhaft, wenn die Steuereinheit dazu ausgebildet ist, ausgehend von dem zweiten Sensorsignal die erste Antriebseinheit derart anzusteuern, dass das Ablageelement um eine vorbestimmte Strecke zum Begrenzungselement hin in die Initialisierungsposition bewegt wird. Dadurch wird eine sichere Bewegung des Ablageelements in die Initialisierungsposition erreicht.

Besonders vorteilhaft ist es, wenn die Vorrichtung ein Verschlusselement umfasst, das das Ausgabefach in einer geschlossenen Position an einer dritten Seite begrenzt und das in einer geöffneten Position Zugriff auf im Ausgabefach bereitgestellte Wertscheine ermöglicht, wobei das Verschlusselement zum Öffnen des Ausgabefachs von der geschlossenen Position in eine geöffnete Position und zum Schließen des Ausgabefachs von der geöffneten Position in die geschlossene Position bewegbar ist. Dadurch wird ein einfacher Zugriff auf die zur Auszahlung bereitgestellten Wertscheine möglich.

Bei einer vorteilhaften Ausführungsform umfasst die Vorrichtung mindestens eine dritte Sensoreinheit zum Erfassen der geschlossenen Position und/oder mindestens eine vierte Sensoreinheit zum Erfassen der geöffneten Position des Verschlusselements. Die dritte Sensoreinheit ist dazu ausgebildet, ein drittes Sensorsignal zu erzeugen und an die Steuereinheit zu übermitteln und/oder die vierte Sensoreinheit ist dazu ausgebildet, ein viertes Sensorsignal zu erzeugen und an die Steuereinheit zu übermitteln. Die Steuereinheit ist dazu ausgebildet, eine zweite Antriebseinheit zum Antrieb des Verschlusselements in Abhängigkeit des dritten Sensorsignals und/oder des vierten Sensorsignals anzusteuern. Dadurch wird eine automatisierte und einfache Steuerung der Bewegung des Verschlusselements erreicht.

Es ist vorteilhaft, wenn die Vorrichtung eine fünfte Sensoreinheit umfasst, die insbesondere in dem Ausgabefach angeordnet ist, und die dazu ausgebildet ist, ein fünftes Sensorsignal zu erzeugen und an die Steuereinheit zu übermitteln, wenn mindestens ein Wertschein in dem Ausgabefach aufgenommen ist. Dadurch wird eine einfache Überwachung des Ausgabefachs erreicht.

Ferner ist es vorteilhaft, wenn mehrere in dem Ausgabefach aufgenommene Wertscheine einen Wertscheinstapel bilden, wobei die Wertscheine des Wertscheinstapels derart übereinander angeordnet sind, dass der unterste Wertschein mit seiner Vorderseite oder seiner Rückseite auf dem Ablageelement liegt und dass der oberste Wertschein mit seiner Vorderseite oder seiner Rückseite das Sensorelement kontaktiert, wobei die Richtung von dem untersten Wertschein zum obersten Wertschein eine Stapelrichtung des Wertscheinstapels ist. Dadurch ist ein sicheres und zuverlässiges Ablegen und Stapeln der Wertscheine möglich.

Bei einer besonders vorteilhaften Ausführungsform detektiert die erste Sensoreinheit die erste Sensorelementposition, wenn der Wertscheinstapel das Sensorelement kontaktiert, wobei das Sensorelement durch den Kontakt mit dem Wertscheinstapel in Richtung des Begrenzungselements bewegt wird. Dadurch wird eine einfache Überwachung der Höhe des Wertscheinstapels erreicht.

Es ist vorteilhaft, wenn die Steuereinheit ausgehend von dem Empfang des ersten Sensorsignals und des fünften Sensorsignals die erste Antriebseinheit derart ansteuert, dass das Ablageelement um eine vorbestimmte Strecke von dem Begrenzungselement weg von der ersten Zuführposition in eine zweite Zuführposition oder von der zweiten Zuführposition in eine dritte Zuführposition bewegt wird, derart, dass weitere Wertscheine in das Ausgabefach zuführbar sind. Dadurch wird eine geordnete Ablage und Stapelung der Wertscheine in dem Ausgabefach sichergestellt.

Besonders vorteilhaft ist es, wenn die Steuereinheit zum Öffnen des Ausgabefachs dazu ausgebildet ist, die erste Antriebseinheit derart anzusteuern, dass das Ablageelement ausgehend von der unteren Position oder von der ersten Zuführposition oder von der zweiten Zuführposition oder von der dritten Zuführposition um eine vorbestimmte Strecke in eine Mitnahmeposition bewegt wird, wobei das Begrenzungselement bei der Bewegung des Ablageelements von einer ersten Begrenzungsposition in eine zweite Begrenzungsposition bewegt wird, und die zweite Antriebseinheit in Abhängigkeit des dritten Sensorsignals zum Bewegen des Verschlusselements von der geschlossenen in die geöffnete Position anzusteuern, wobei ein Eingriffselement des Begrenzungselements mit einem Eingriffsbereich des Verschlusselements in Eingriff gelangt. Dadurch wird erreicht, dass das Volumen des Ausgabefachs beim Öffnen auf besonders einfache Weise vergrößert wird, sodass ein besonders einfacher Zugriff auf die zur Auszahlung bereitgestellten Wertscheine möglich ist.

Es ist vorteilhaft, wenn die erste Sensoreinheit und/oder die zweite Sensoreinheit und/oder die dritte Sensoreinheit und/oder die vierte Sensoreinheit und/oder die fünfte Sensoreinheit mindestens ein Detektionselement umfasst, wobei das Detektionselement das Sensorsignal erzeugt und an die die Steuereinheit überträgt, und wobei das Detektionselement vorzugsweise eine Lichtschranke umfasst. Dadurch wird erreicht, dass die Sensoreinheiten einfach aufgebaut und kostengünstig sind.

Ferner ist es vorteilhaft, wenn die Vorrichtung mindestens eine dritte Antriebseinheit zum Antrieb von Transportelementen zum Transport der Wertscheine umfasst, wobei die Transportelemente zum Zuführen der Wertscheine in das Ausgabefach in eine erste Richtung antreibbar sind und wobei die Transportelemente in eine zweite, der ersten Richtung entgegengesetzte Richtung zum Transport der Wertscheine aus dem Ausgabefach heraus in Richtung einer Wertscheinhandhabungseinheit der Vorrichtung antreibbar sind. Dadurch wird eine kompakte Bauweise der Vorrichtung erreicht.

Besonders vorteilhaft ist es, wenn die Vorrichtung eine sechste Sensoreinheit zur Detektion mindestens eines Merkmals der auszuzahlenden Wertscheine umfasst, wobei die Steuereinheit dazu ausgebildet ist, in Abhängigkeit des durch die sechste Sensoreinheit detektierten Merkmals zu unterscheiden, ob der Wertschein ein für die Auszahlung zulässiger Wertschein ist, und wenn die Steuereinheit die dritte Antriebseinheit in die erste Richtung antreibt, wenn die sechste Sensoreinheit einen zur Auszahlung zulässigen Wertschein detektiert. Dadurch wird erreicht, dass ausschließlich Wertscheine, die zur Auszahlung zulässig sind, dem Ausgabefach zugeführt werden.

Bei einer vorteilhaften Ausführungsform ist die Steuereinheit dazu ausgebildet, die zweite Antriebseinheit derart anzusteuern, dass das Verschlusselement zur Entnahme der Wertscheine für einen vorbestimmten Zeitraum in der geöffneten Position angeordnet ist und nach dem voreingestellten Zeitraum in die geschlossene Position bewegt wird. Ferner ist die Steuereinheit dazu ausgebildet, zu prüfen, ob die fünfte Sensoreinheit nach Ablauf des voreingestellten Zeitraums das fünfte Sensorsignal weiter zur Steuereinheit überträgt. Die Steuereinheit ist, falls die Prüfung ergibt, dass das fünfte Sensorsignal weiter zur Steuereinheit übertragen wird, dazu ausgebildet, die erste Antriebseinheit derart anzusteuern, dass das Ablageelement von der Mitnahmeposition in eine Abzugsposition bewegt wird, und die dritte Antriebseinheit derart anzusteuern, dass die Transportelemente in die zweite Richtung angetrieben werden. Dadurch wird erreicht, dass Wertscheine, die nicht aus dem Ausgabefach entnommen werden, sicher als sogenannte Retracts aus dem Ausgabefach heraus, vorzugsweise in einen Retract-Rejectbehälter, transportiert werden.

Weitere Merkmale und Vorteile ergeben sich aus der folgenden Beschreibung, welche in Verbindung mit den beigefügten Figuren Ausführungsbeispiele näher erläutert.

Es zeigt:
- Fig. 1: eine schematische Darstellung einer Vorrichtung zur Handhabung von Wertscheinen,
- Fig. 2: eine perspektivische Detailansicht einer Wertscheinausgabeeinheit gemäß einer ersten Ausführungsform mit einem geschlossenen Verschlusselement zum Einsatz in der Vorrichtung zur Handhabung von Wertscheinen nach Figur 1,
- Fig. 3: eine Schnittdarstellung eines Ausschnitts der Wertscheinausgabeeinheit nach Figur 2,
- Fig. 4: eine perspektivische Detailansicht der Wertscheinausgabeeinheit nach den Figuren 2 und 3 mit geöffnetem Verschlusselement,
- Fig. 5: einen Ausschnitt einer perspektivischen Schnittdarstellung der Wertscheinausgabeeinheit nach Figur 4,
- Fig. 6: eine perspektivische Ansicht eines Begrenzungselements der Wertscheinausgabeeinheit nach den Figuren 2 bis 5,
- Fig. 7: eine perspektivische Schnittdarstellung einiger Elemente der Wertscheinausgabeeinheit nach den Figuren 2 bis 6,
- Fig. 8: eine vergrößerte Detailansicht eines Eingriffselements nach Figur 7,
- Fig. 9: eine perspektivische Detailansicht eines Ablageelements und des Begrenzungselements der Wertscheinausgabeeinheit nach den Figuren 2 bis 8,
- Fig. 10: eine weitere perspektivische Detailansicht der Wertscheinausgabeeinheit nach den Figuren 2 bis 9,
- Fig. 11: eine weitere perspektivische Detailansicht der Wertscheinausgabeeinheit nach den Figuren 2 bis 10,
- Fig. 12: eine weitere schematische Darstellung der Wertscheinausgabeeinheit nach den Figuren 2 bis 11,
- Fig. 13: eine perspektivische Ansicht einer Wertscheinausgabeeinheit gemäß einer zweiten Ausführungsform, die zum Einsatz in der Vorrichtung nach Figur 1 alternativ zu der Wertscheinausgabeeinheit nach den Figuren 2 bis 12 geeignet ist,
- Fig. 14: eine perspektivische Detailansicht eines Ausschnitts der Wertscheinausgabeeinheit nach Figur 13,
- Fig. 15: den Ausschnitt der Wertscheinausgabeeinheit nach Figur 14, wobei ein Begrenzungselement der Wertscheinausgabeeinheit ausgeblendet ist,
- Fig. 16: eine perspektivische Detailansicht des Ablageelements und von Andruckelementen der Wertscheinausgabeeinheit nach den Figuren 13 bis 15,
- Fig. 17: eine weitere perspektivische Detailansicht des Ablageelements nach Figur 16 ohne Andruckelemente,
- Fig. 18: eine weitere perspektivische Detailansicht der Andruckelemente nach Fig. 16,
- Fig. 19: ein Ablaufplan eines Verfahrens zur Initialisierung einer Wertscheinausgabeeinheit nach einer der Figuren 2 bis 18, und
- Fig. 20: Signaldiagramm zur Steuerung der Initialisierung gemäß dem Ablauf nach Figur 19.

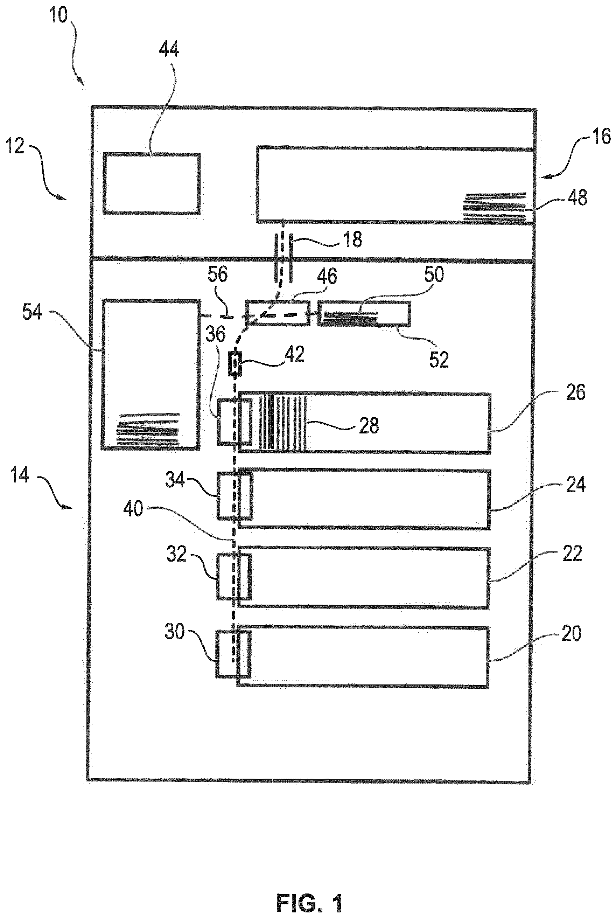

Figur 1 zeigt eine schematische Darstellung einer Vorrichtung 10 zur Handhabung von Wertscheinen 28, 48, 50. Die Vorrichtung 10 umfasst ein Kopfmodul 12 und ein Tresormodul 14. Das Kopfmodul 12 umfasst eine Wertscheinausgabeeinheit 16 zur Ausgabe von auszuzahlenden Wertscheinen 48 an eine Bedienperson. Über eine Übergabeöffnung 18 werden Wertscheine 28, 48 von dem Tresormodul 14 in das Kopfmodul 12 transportiert. Das Tresormodul 14 umfasst vier Wertscheinkassetten 20 bis 26, die zur Aufbewahrung und zum Transport von Wertscheinen 28 dienen.

Die Wertscheine 28 sind in den Wertscheinkassetten 20 bis 26 in Form eines Stapels abgelegt. Einer dieser Stapel ist in der ersten Wertscheinkassette 26 beispielhaft angedeutet. Einer der Wertscheine dieses Wertscheinstapels ist beispielhaft mit dem Bezugszeichen 28 bezeichnet. Bei der in Figur 1 gezeigten Betriebsposition der Wertscheinkassetten 20 bis 26 sind die Wertscheine 28 in den Wertscheinkassetten 20 bis 26 auf einer ihrer Kanten stehend angeordnet. Jede Wertscheinkassette 20 bis 26 hat jeweils eine nicht dargestellte Öffnung zur Entnahme von Wertscheinen 28. Vor der Öffnung ist eine Vereinzelungseinheit 30 bis 36 angeordnet, mit deren Hilfe die in den Wertscheinkassetten 20 bis 26 aufbewahrten Wertscheine 28 einzeln aus den Wertscheinkassetten 20 bis 26 entnommen und dadurch vereinzelt werden können.

Sobald ein Benutzer einen Auszahlungsvorgang initiiert, werden auszuzahlende Wertscheine 28 aus den entsprechenden Wertscheinkassetten 20 bis 26 entnommen und nach der Vereinzelung einem Transportpfad 40 zugeführt. Eine Sensoreinheit 42 ist entlang des Transportpfads 40 angeordnet und detektiert mindestens ein Merkmal jedes auszuzahlenden Wertscheins 28. Die Sensoreinheit 42 übermittelt einen Messwert des detektieren Merkmals an eine Steuereinheit 44, die die dazu ausgebildet ist, in Abhängigkeit des detektieren Merkmals zu ermitteln, ob der Wertschein 28 ein für eine Auszahlung zulässiger erster Wertschein 48 oder ein für die Auszahlung nicht zulässiger zweiter Wertschein 50 ist.

Die Steuereinheit 44 steuert eine Weicheneinheit 46 derart an, dass der Wertschein 48 der Wertscheinausgebeinheit 16 zugeführt wird, wenn er ein für die Auszahlung zulässiger Wertschein 48 ist. Ist der Wertschein jedoch ein für die Auszahlung nicht zulässiger Wertschein 50, steuert die Steuereinheit 44 die Weicheneinheit 46 derart an, dass der Wertschein 50 einem Zwischenspeicher 52 zugeführt wird.

Um den Auszahlungsvorgang für die Bedienperson nicht unnötig zu verzögern, werden für die nicht zur Auszahlung zulässigen Wertscheine 50 Ersatzwertscheine aus den Wertscheinkassetten 20 bis 26 entnommen und, sofern diese aufgrund der mit Hilfe der Sensoreinheit 42 detektierten Merkmale für die Auszahlung zulässig sind, zur Wertscheinausgabeeinheit 16 transportiert. Somit kann der gewünschte Gesamtbetrag ohne Unterbrechung des Auszahlungsvorgangs an die Bedienperson ausgegeben werden. Nach dem Abschluss des Auszahlungsvorgangs werden die nicht zur Auszahlung zulässigen Wertscheine 50 über einen weiteren Transportpfad 56 einem Behälter 54, insbesondere einem sogenannten Reject-und Retractbehälter 54, zugeführt, der vorzugsweise in Form einer weiteren Wertscheinkassette ausgebildet ist.

Der Reject- und Retractbehälter 54 umfasst insbesondere ein erstes Fach zur Aufnahme der aussortierten Wertscheine 50 (sogenannten Rejects) und ein von dem ersten Fach getrenntes zweites Fach zur Aufnahme von dem Ausgabefach nicht entnommenen Wertscheinen 48 (sogenannte Retracts). Somit können die Rejects und Retracts getrennt gespeichert werden, wodurch eine Notenrückverfolgung einfach möglich ist.

Bei einer alternativen Ausführungsform kann die Vorrichtung 10 als Reyclinggeldautomat ausgebildet sein, d.h. als Vorrichtung 10 zum Auszahlen und Einzahlen von Wertscheinen 28. Bei dieser Ausführungsform prüft die Sensoreinheit 42 oder eine weitere, insbesondere im Kopfmodul angeordnete Sensoreinheit auch die Merkmale der Wertscheine 28, die eine Bedienperson über eine Wertscheinausgabe- und Eingabeeinheit der Vorrichtung 10 zuführt. Zur Einzahlung zulässige Wertscheine 28 werden den Wertscheinkassetten 20 bis 26 zugeführt, zur Einzahlung nicht zulässige Wertscheine werden dem Zwischenspeicher 52 zugeführt. Bei anderen Ausführungsformen kann die Vorrichtung 10 mehr oder weniger Wertscheinkassetten 20 bis 26 umfassen, sowie getrennte Reject- und Retractbehälter 54.

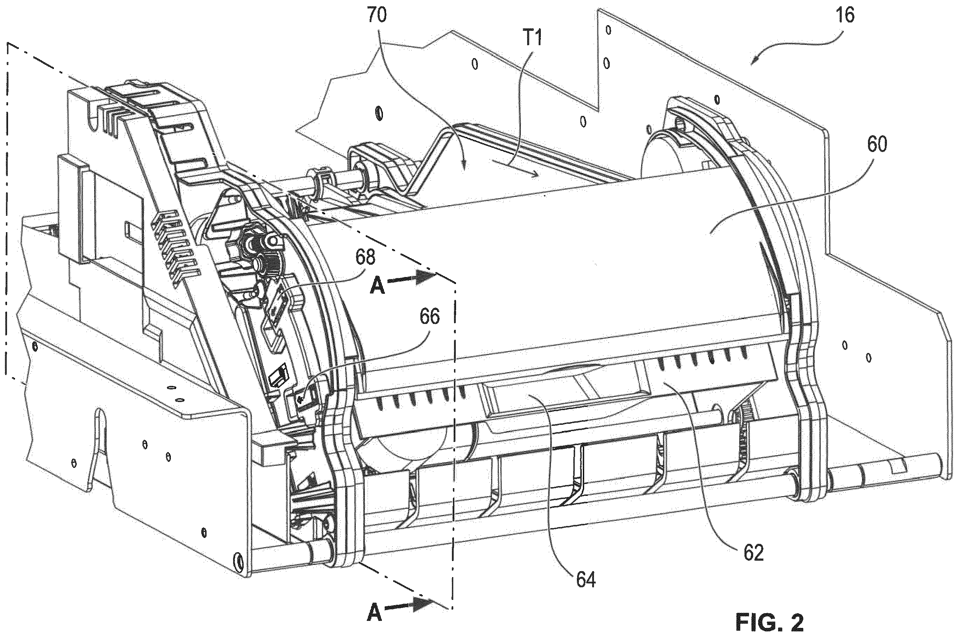

Figur 2 zeigt eine perspektivische Detailansicht der Wertscheinausgabeeinheit 16 der Vorrichtung 10 mit einem geschlossenen Verschlusselement 60. Element mit gleichem Aufbau oder gleicher Funktion haben dieselben Bezugszeichen. Das Verschlusselement 60 begrenzt ein in der Darstellung nach Figur 2 von dem Verschlusselement 60 verdecktes Ausgabefach, in dem die zur Auszahlung bestimmten Wertscheine 48 zur Entnahme durch eine Bedienperson bereitgestellt werden.

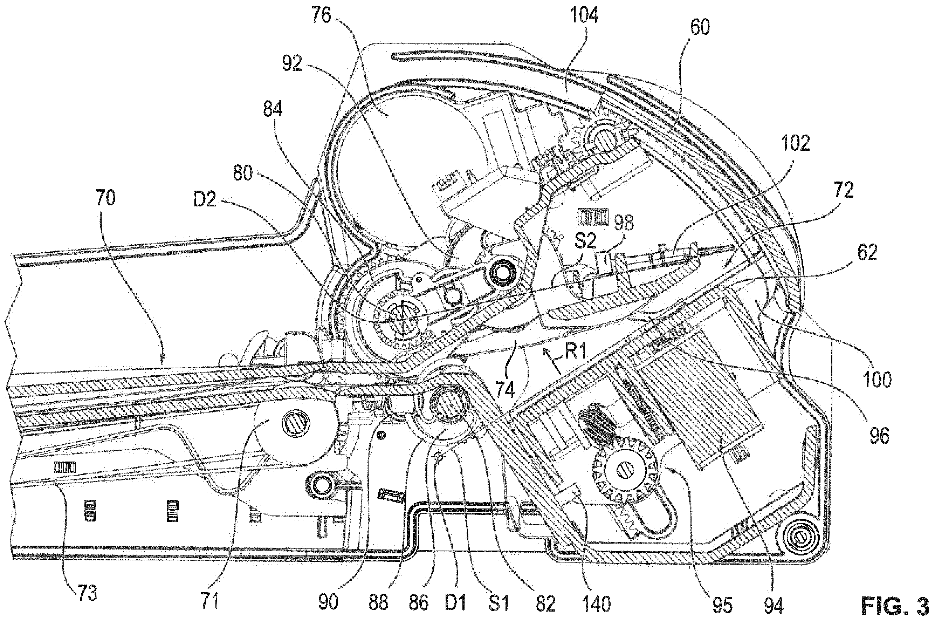

Das Ausgabefach ist in Figur 3 bezeichnet und wird nachfolgend noch näher beschrieben. Bei der in Figur 2 dargestellten geschlossenen Position des Verschlusselements 60 ist ein Zugriff auf das Ausgabefach 72 und auf darin bereitgestellte Wertscheine 48 nicht möglich.

Bei der Darstellung nach Figur 2 ist ein Ablageelement 62 zur Ablage der zur Auszahlung bereitgestellten Wertscheine 48 sichtbar. Das Ablageelement 62 umfasst eine Aussparung 64, die derart angeordnet ist, dass die in dem Ausgabefach 72 abgelegten Wertscheine 48 über einen Teilbereich der Aussparung 64 liegen. Bei geöffneter Position des Verschlusselements 60 wird der Zugriff auf die Wertscheine 48 dadurch erleichtert, dass eine Bedienperson mit ihren Fingern in die Aussparung 64 unter die Wertscheine 48 greifen kann.

In Figur 2 sind ferner zwei Sensoreinheiten 66, 68 sichtbar, wobei die Sensoreinheit 66 zum Erfassen der geschlossenen Position des Verschlusselements 60 und die Sensoreinheit 68 zum Erfassen einer geöffneten Position des Verschlusselements 60 vorgesehen ist. Die Sensoreinheiten 66, 68 erzeugen beim Erfassen der jeweiligen Position des Verschlusselements 60 ein Sensorsignal und übermitteln dieses Sensorsignal an die Steuereinheit 44. Die Steuereinheit 44 ist dazu ausgebildet, eine in Figur 2 nicht sichtbare Antriebseinheit zum Bewegen des Verschlusselements 60 in Abhängigkeit des übermittelten Sensorsignals anzusteuern.

Die Wertscheinausgabeeinheit 16 umfasst ferner eine Transporteinheit 70, mit deren Hilfe die aus dem Tresormodul 14 entnommenen Wertscheine 48 in eine Richtung T1 zum Ausgabefach 72 transportiert werden. Die aus dem Ausgabefach 72 nicht entnommenen Wertscheine werden mit Hilfe der Transporteinheit 70 in eine der Richtung T1 entgegengesetzte Richtung transportiert.

Das Verschlusselement 60 ist ein erstes inneres Verschlusselement der Vorrichtung 10, das als "Schatten"-Verschlusselement 60 hinter einem zweiten, nicht dargestellten äußeren Verschlusselement angeordnet ist. Das äußere Verschlusselement kann unabhängig von dem Verschlusselement 60 geöffnet und geschlossen werden. Somit ist die Entnahme von Wertscheinen 48 aus der Wertscheinausgabeeinheit 16 nur bei gleichzeitigem Öffnen beider Verschlusselemente 60 möglich. Dadurch wird ein besonders hohes Maß an Sicherheit erreicht, da insbesondere bei einer Manipulation des äußeren Verschlusselements durch unbefugte Dritte das "Schatten"-Verschlusselement 60 verschlossen bleibt.

Figur 3 zeigt eine Schnittdarstellung eines Ausschnitts der Wertscheinausgabeeinheit 16 nach Figur 2 entlang der Schnittebene A. Bei einem Auszahlungsvorgang werden die Wertscheine 48 zunächst über zwei jeweils über zwei Umlenkrollen umgelenkte Transportriemen in Richtung des Ausgabefachs 72 transportiert, wobei bei der Schnittdarstellung nach Figur 3 der Transportriemen 73 und die Umlenkrolle 71 sichtbar sind. Anschließend werden die Wertscheine 48 weiteren Transportelementen 80 bis 90 zugeführt, mit deren Hilfe die Wertscheine 48 dem Ausgabefach 72 vorzugsweise einzeln zugeführt werden. Die Transportelemente 80 bis 90 umfassen Transportrollen, Flügelräder und Andruckrollen, wobei in der Darstellung nach Figur 3 die auf der Welle 80 angeordnete Transportrolle 84, die auf der Welle 82 angeordnete Transportrolle 86 und das Flügelrad 88, sowie die Andruckrolle 90 sichtbar sind. Die Wellen 80 und 82 sind über eine in Figur 3 nicht sichtbare Getriebeanordnung miteinander verbunden und werden mit Hilfe der Antriebseinheit 76 angetrieben.

Beim Zuführen der Wertscheine 48 in das Ausgabefach 72 wird die Antriebseinheit 76 von der Steuereinheit 44 derart angesteuert, dass die Wellen 80 und 82 jeweils in einer ersten Drehrichtung angetrieben werden. Insbesondere dreht sich die Welle 80 entgegen dem Uhrzeigersinn und die Welle 82 entsprechend im Uhrzeigersinn, sodass der Wertschein 48 dem Ausgabefach 72 zugeführt wird.

Werden die Wertscheine 48 nicht aus der Vorrichtung 10 entnommen, so werden die sogenannten Retracts aus dem Ausgabefach 72 heraus dem Reject-und Retractbehälters 54 zugeführt. Die Entnahme erfolgt mit Hilfe von Abzugsrollen 92, die sicherstellen, dass die Wertscheine 48 vorzugsweise einzeln oder alternativ als Stapel aus dem Ausgabefach 72 entnommen werden. Bei der Entnahme der Wertscheine aus dem Ausgabefach 72 werden die Wellen 80 und 82 jeweils in einer zweiten Drehrichtung angetrieben. Insbesondere dreht sich die Welle 80 im Uhrzeigersinn und die Welle 82 entsprechend gegen den Uhrzeigersinn, um den Wertschein 48 aus dem Ausgabefach 72 zu entnehmen.

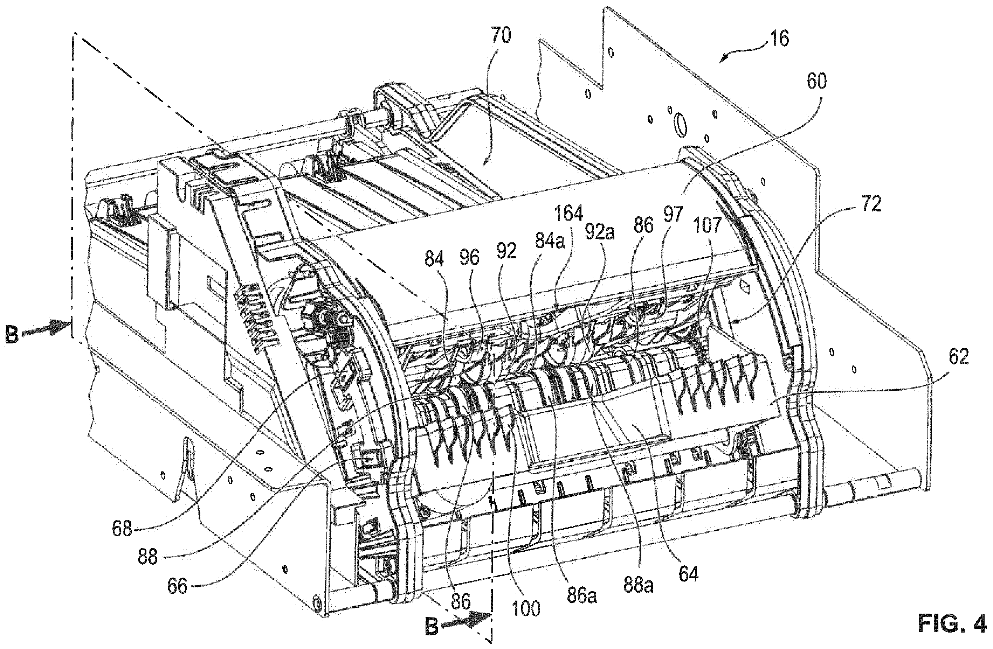

Das Ausgabefach 72 wird auf einer ersten Seite von dem Ablageelement 62 und auf einer gegenüberliegenden zweiten Seite von einem Begrenzungselement 74 begrenzt. Das Begrenzungselement 74 umfasst zwei Andruckelemente 96, 97 zur geordneten Ablage der Wertscheine 48, wobei bei der Darstellung nach Figur 3 nur das Andruckelement 96 sichtbar ist, während das zweite Andruckelement 97 in den Figuren 4, 6, 9 und 10 dargestellt ist. Die nachfolgende Beschreibung für das Andruckelement 96 gilt entsprechend auch für das Andruckelement 97, das den selben Aufbau und dieselbe Funktion des Andruckelements 96 hat.

Das Andruckelement 96 ist vorzugsweise schwenkbar an dem Begrenzungselement 76 angeordnet und mit diesem verbindbar. Bei der Darstellung nach Figur 3 sind keine Wertscheine 48 im Ausgabefach 72 abgelegt. Das Begrenzungselement 74 befindet sich in einer ersten Begrenzungsposition und das Andruckelement 96 befindet sich in einer ersten Andruckposition, wobei das Andruckelement 96 das Ablageelement 62 kontaktiert. Wird dem Ausgabefach 72 ein Wertschein 48 zugeführt, wird die Bewegung des Wertscheins 48 durch das Andruckelement 96 abgebremst bzw. gestoppt. Zudem lenkt der zugeführte Wertschein 48 das Andruckelement 96 in Richtung des Begrenzungselements 74 aus. Das Andruckelement 96 drückt den zugeführten Wertschein 48 schon beim Zuführen in das Ausgabefach 72 gegen das Ablageelement 62 und stellt dadurch eine geordnete Ablage der Wertscheine sicher.

Die zugeführten Wertscheine 48 bilden einen Wertscheinstapel, wobei die Wertscheine 48 des Wertscheinstapels derart übereinander angeordnet sind, dass der unterste, d.h. der erste zugeführte Wertschein 48, mit seiner Vorderseite oder seiner Rückseite auf dem Ablageelement 62 liegt. Der oberste Wertschein 48 kontaktiert mit seiner Vorderseite oder seiner Rückseite das Andruckelement 96. Die Richtung von dem untersten Wertschein 48 zum obersten Wertschein 48 ist eine Stapelrichtung R1 des Wertscheinstapels. Je mehr Wertscheine 48 auf dem Ablageelement 62 abgelegt werden, desto höher ist der von den Wertscheinen 48 gebildete Wertscheinstapel und desto stärker ist die Auslenkung des Andruckelements 96 in Richtung des Begrenzungselements 74. Bei der Darstellung nach Figur 3 ist eine Sensoreinheit 98 sichtbar, die dazu ausgebildet ist, eine zweite Andruckposition des Andruckelements 96 zu erfassen, die einer voreingestellten Auslenkung des Andruckelements 96 und somit einer voreingestellten Höhe des Wertscheinstapels entspricht.

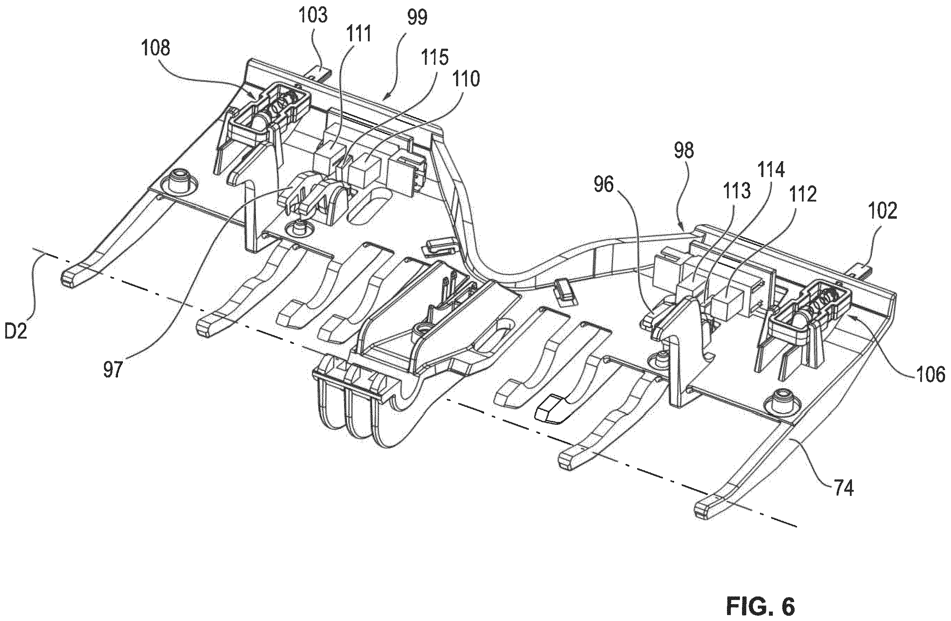

Die Sensoreinheit 98 umfasst vorzugsweise eine Lichtschranke mit einem Sender und mit einem Empfänger, die gegenüberliegend angeordnet sind. Die Sensoreinheit 98, die dem Andruckelement 96 zugeordnet ist, und eine weitere Sensoreinheit 99, die dem Andruckelement 97 zugeordnet ist, werden im Zusammenhang mit Figur 6 noch näher beschrieben. Bei dem Auslenken des Andruckelements 96 in die zweite Andruckposition unterbricht ein in Figur 3 nicht sichtbares Unterbrecherelement des Andruckelements 96 den vom Sender der Sensoreinheit 98 ausgesandten Lichtstrahl, wobei der Empfänger die Unterbrechung detektiert. Sobald die Unterbrechung des Lichtstrahls der Sensoreinheit 98 bzw. der Lichtstrahlen beider Sensoreinheiten 98, 99 von der Sensoreinheit 98 detektiert wird, erzeugt diese ein Detektionssignal und übermittelt das Detektionssignal an die Steuereinheit 44. Daraufhin steuert die Steuereinheit 44 eine Antriebseinheit 94 an, die das Ablageelement 62 über eine Getriebeanordnung 95 um einen voreingestellten Abstand entgegen der Stapelrichtung R1 bewegt.

Durch diese Bewegung wird das Andruckelement 96 aus der zweiten Andruckposition von dem Begrenzungselement 74 weg bewegt, sodass die zweite Andruckposition des Andruckelements 96 von der Sensoreinheit 98 nicht mehr erfasst wird. Daraufhin können weitere Wertscheine 48 dem Ausgabefach 72 zugeführt werden, wobei der oben beschriebene Vorgang der Bewegung des Ablageelements 62 entgegen der Richtung R1 wiederholt wird, wenn das Andruckelement 96 von den abgelegten Wertscheinen 48 ein weiteres Mal in die zweite Andruckposition bewegt wird.

Das Ablageelement 62 umfasst mehrere Rippenelemente, von denen in der Darstellung nach Figur 3 ein mit dem Bezugszeichen 100 bezeichnetes Rippenelement sichtbar ist. Bei der in Figur 3 dargestellten geschlossenen Position des Verschlusselements 60 greift das Rippenelement 100 in eine komplementäre Aussparung des Verschlusselements 60. Dadurch wird ein Zugriff auf das Ausgabefach 72 durch unbefugte Dritte erschwert. Das Begrenzungselement 74 umfasst ferner ein Eingriffselement 102, das zwischen einer in Figur 3 dargestellten ausgefahrenen Position und einer eingefahrenen Position bewegbar ist.

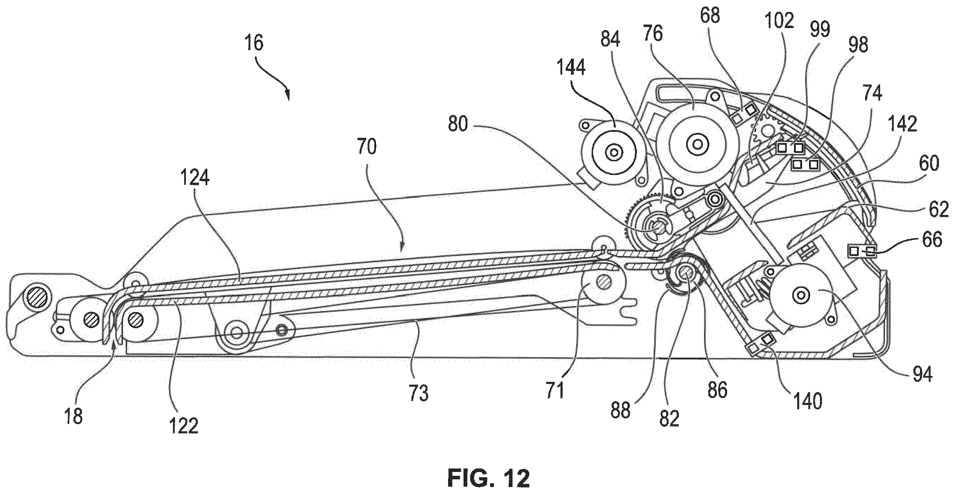

Sobald alle zur Auszahlung bestimmten Wertscheine 48 dem Ausgabefach 72 zugeführt worden sind, führt die Steuereinheit 44 Schritte zum Öffnen des Ausgabefachs 72 aus, die nachfolgend im Zusammenhang mit Figur 12 noch im Detail beschrieben werden Dabei wird das Ablageelement 62 mit Hilfe der Antriebseinheit 94 in Richtung des Begrenzungselements 74 bewegt, wobei das Begrenzungselement 74 derart angeordnet ist, dass es bei dieser Bewegung des Ablageelements 62 in eine zweite Begrenzungsposition bewegt wird. Ferner steuert die Steuereinheit eine weitere in der Darstellung nach Figur 3 nicht sichtbare Antriebseinheit 104 zum Bewegen des Verschlusselements 60 von der in Figur 3 dargestellten geschlossenen Position in eine geöffnete Position an. Während der Bewegung von der geschlossenen in die geöffnete Position gelangt das Verschlusselement 60 in Eingriff mit dem Eingriffselement 102 des in der zweiten Begrenzungsposition angeordneten Begrenzungselements 74.

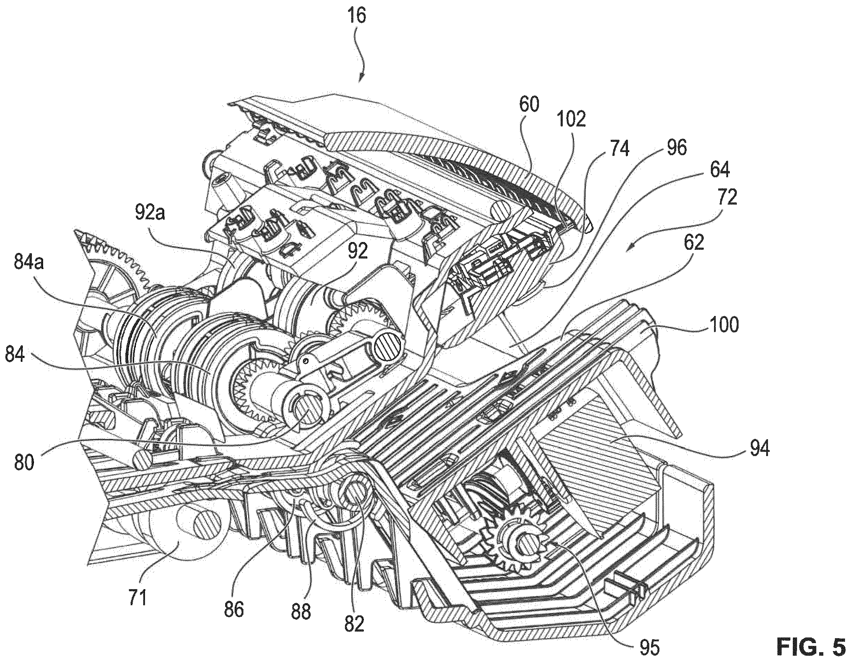

Nach dem Eingriff mit dem Verschlusselement 60 werden das Eingriffselement 102 und das Begrenzungselement 74 gemeinsam mit dem Verschlusselement 60 bewegt, bis das Verschlusselement 60 die in den Figuren 4 und 5 dargestellte geöffnete Position einnimmt. Im Zuge dieser gemeinsamen Bewegung des Begrenzungselements 74 mit dem Verschlusselement 60 wird das Begrenzungselement 74 von der zweiten Begrenzungsposition in eine dritte, geöffnete Begrenzungsposition bewegt.

Während der Bewegung von der geschlossenen Position in die geöffnete Position wird das Verschlusselement 60 in einer Kulissenbahn geführt, wobei insbesondere eine Bewegung des Verschlusselements 60 um eine virtuelle Drehachse D1 erfolgt. Das Begrenzungselement 74 wird hingegen um eine zweite, von der Drehachse D1 verschiedene Drehachse D2 gedreht, die mit der Längsachse der Welle 80 übereinstimmt. Somit ist der Schwenkradius S1 des Verschlusselements 60 größer als der Schwenkradius S2 des Begrenzungselements 74. Durch die beschriebene Anordnung des Verschlusselements 60 und des Begrenzungselements 74 wird erreicht, dass das Eingriffselement 102 von der ausgefahrenen in die eingefahrene Position bewegt wird, während das Begrenzungselements 74 von der zweiten Begrenzungsposition in die dritte Begrenzungsposition bewegt wird. Dabei verformt das Eingriffselement 102 ein in der Darstellung nach Figur 3 nicht sichtbares elastisches Element, vorzugsweise eine Rückstellfeder.

Ferner ist die Steuereinheit 44 dazu ausgebildet, während der Bewegung des Begrenzungselements 74 von der zweiten Begrenzungsposition in die dritte Begrenzungsposition, die Antriebseinheit 94 zum Bewegen des Ablageelements 62 in die dem Begrenzungselement 74 entgegengesetzte Richtung anzusteuern. Dadurch wird erreicht, dass das Volumen des Ausgabefachs 72 bei geöffnetem Verschlusselement 60 weiter vergrößert wird, so dass ein leichter Zugriff auf den im Ausgabefach 62 befindlichen Wertschein 48 oder Wertscheinstapel möglich ist. Die Position des Ablageelements wird mit Hilfe einer Sensoreinheit 140 überwacht, wobei die Steuereinheit dazu ausgebildet ist, in Abhängigkeit eines Signals der Sensoreinheit 140, die Antriebseinheit 94 anzusteuern.

Figur 4 zeigt eine perspektivische Detailansicht der Wertscheinausgabeeinheit 16 nach den Figuren 2 und 3 mit geöffnetem Verschlusselement 60. Bei der Darstellung nach Figur 4 sind zusätzlich zu den in Figur 3 dargestellten Transportelementen auch die Transportrollen 84a und 86a, die Flügelräder 88a und 88b, sowie die Abzugsrolle 92a und das zweite Ablageelement 97 sichtbar. Außerdem ist ein Teil der Getriebeanordnung 106 sichtbar. Ferner ist eine zweite Aussparung 164 sichtbar, die der Aussparung 64 gegenüberliegend angeordnet ist und den Zugriff auf die Wertscheine 48 dadurch weiter erleichtert, dass eine Bedienperson mit ihren Fingern in die Aussparung 64 unter die Wertscheine 48 und in die Aussparung 164 von oben auf die Wertscheine 48 greifen kann. Bei der in Figur 4 gezeigten geöffneten Position des Verschlusselements 60 ist ein Zugriff auf im Aufnahmefach 72 abgelegte Wertscheine 48 einfach möglich, wenn gleichzeitig auch das äußere, nicht dargestellte Verschlusselement geöffnet ist.

Figur 5 zeigt einen Ausschnitt einer perspektivischen Schnittdarstellung der Wertscheinausgabeeinheit 16 entlang der Schnittebene B nach Figur 4. Bei der Darstellung nach Figur 5 ist insbesondere der Eingriff zischen dem Eingriffselement 102 und dem Verschlusselement 60 sichtbar, über welchen das Begrenzungselement 74 in der dritten Begrenzungsposition gehalten wird.

Figur 6 zeigt eine perspektivische Ansicht des Begrenzungselements 76 nach den Figuren 2 bis 5. Das Andruckelement 96 ist zu Darstellungszwecken in der ersten Andruckposition, das Andruckeckelement 97 in der zweiten Andruckposition dargestellt. Während eines Auszahlungsvorgangs nehmen die Andruckelemente 96, 97 jedoch immer eine übereinstimmende Andruckposition ein. Ein Unterbrecherelement 115 des Andruckelements 97 unterbricht den von dem Sender 110 der Sensoreinheit 99 ausgesandten Lichtstrahl, der von dem Empfänger 111 somit nicht mehr detektiert wird, sodass die zweite Andruckposition des Andruckelements 97 von der Steuereinheit 44 detektiert wird. Bei der ersten Andruckposition des Andruckelements 96 wird der Lichtstrahl zwischen dem Sender 112 und dem Empfänger 113 hingegen nicht von dem Unterbrechungselement 114 unterbrochen. Ferner sind bei der Darstellung nach Figur 6 die Federn 106 und 108 sichtbar, die von den jeweiligen Eingriffselementen 102 und 103 elastisch verformt werden, wenn diese von der ausgefahrenen in die eingefahrene Position bewegt werden.

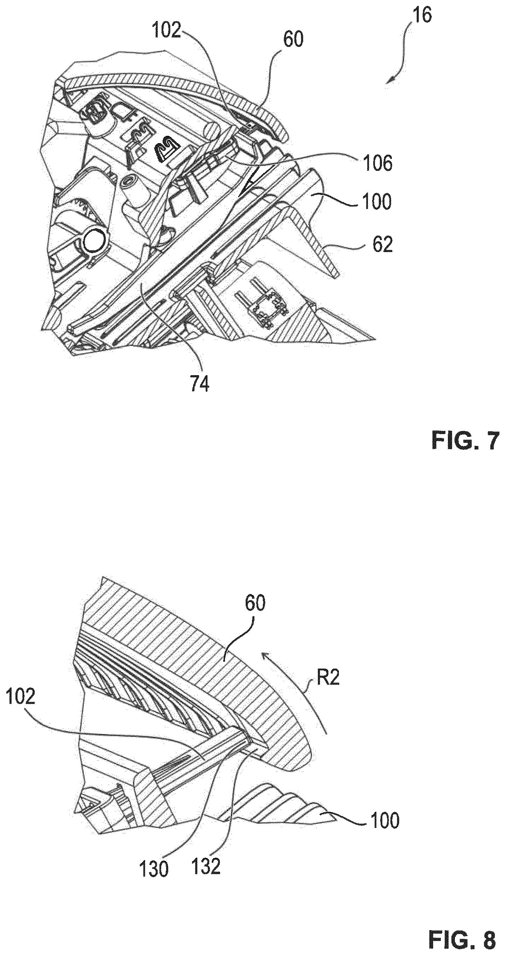

Figur 7 zeigt eine perspektivische Schnittdarstellung einiger Elemente der Wertscheinausgabeeinheit 16 nach den Figu-ren 2 bis 6, bei der sich das Begrenzungselement 74 in der zweiten Begrenzungsposition und sich das Verschlusselement 60 zwischen der geschlossenen Position und der geöffneten Position befindet.

Figur 8 zeigt eine vergrößerte Detailansicht eines Teils des Begrenzungselements 76, des Eingriffselements 102 und eines Teils des Verschlusselements 60 nach Figur 7. Bei der Darstellung nach Figur 8 ist das Begrenzungselement 74 in der zweiten Begrenzungsposition angeordnet. Eine in Richtung des Verschlusselements 60 aus dem Begrenzungselement 74 vorstehende Spitze 130 des Eingriffselements 102 ragt in die Bewegungsbahn eines Vorsprungs 132 des Verschlusselements 60. Bei einer weiteren Bewegung des Verschlusselements 60 in Richtung R2 gelangen der Vorsprung 132 und die Spitze 130 miteinander derart in Eingriff, dass das Eingriffselement 102 von dem Verschlusselement 60 mitgenommen wird, so dass das Begrenzungselement 74 zusammen mit dem Eingriffselement 102 um seine Drehachse D2 verschwenkt wird.

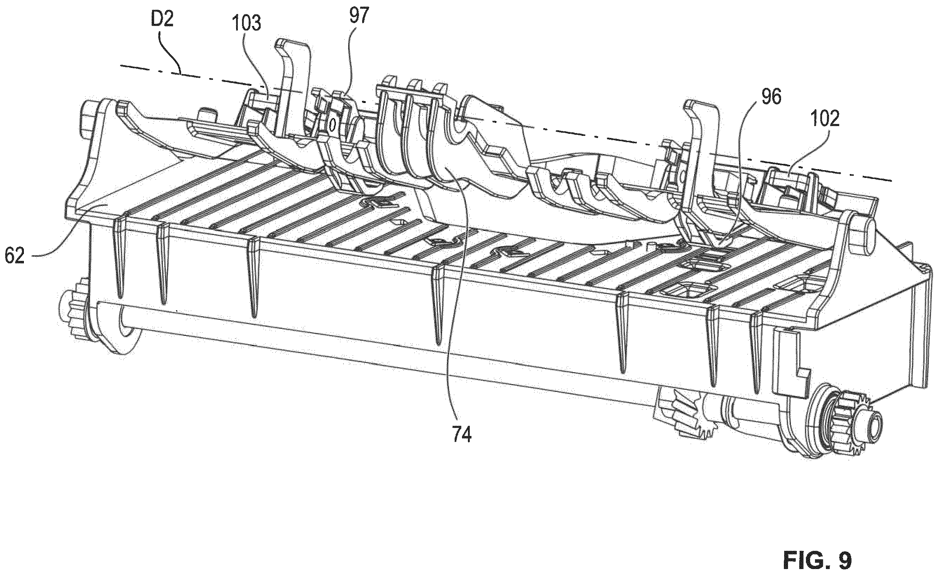

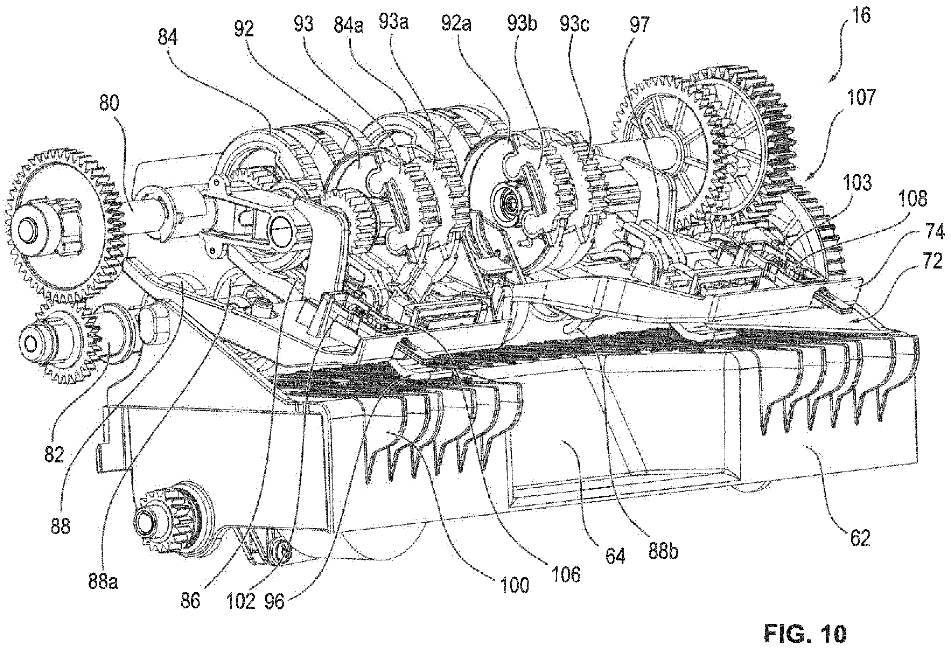

Figur 9 zeigt eine perspektivische Detailansicht des Ablageelements 62 und des Begrenzungselements 74. Figur 10 zeigt eine weitere perspektivische Detailansicht der Wertscheinausgabeeinheit 16. Bei der Darstellung nach Figur 10 sind insbesondere auf den Abzugsrollen 92, 92a angeordnete Abzugsmittel 93, 93a, 93b, 93c sichtbar, die vorzugsweise aus einem Elastomer gefertigt sind und die Vereinzelung der als Retracts klassifizierten, nicht entnommenen Wertscheine 48 aus dem Ausgabefach 72 sicherstellen.

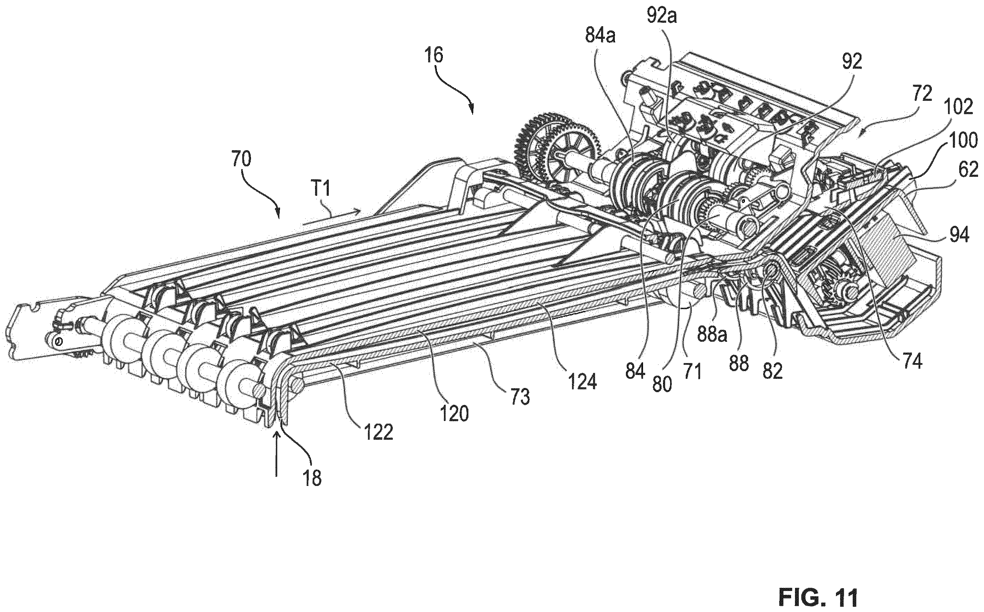

Figur 11 zeigt eine weitere perspektivische Detailansicht der Wertscheinausgabeeinheit 16 nach den Figuren 2 bis 10 mit der Transporteinheit 70 zum Zuführen der Wertscheine 48 in das Ausgabefach 72 und zum Transport der nicht entnommenen Wertscheine 48 in Richtung des Reject- und Retractbehälters 54. Bei dem Auszahlungsvorgang werden die Wertscheine 48 über die Übergabeöffnung 18 aus dem Tresormodul 14 der Transporteinheit 70 zugeführt und in Richtung T1 bis zum Ausgabefach 72 transportiert. Nicht entnommene Wertscheine 48 werden in der der Richtung T1 entgegengesetzten Richtung T2 aus dem Ausgabefach 72 heraus transportiert. Der Transportpfad 120 der Wertscheine 48 wird an einer ersten Seite von dem Transportriemen 73 und einem Bodenelement 122 und auf der gegenüberliegenden Seite von einem Abdeckelement 124 begrenzt.

Figur 12 zeigt eine weitere schematische Darstellung der Wertscheinausgabeeinheit 16 nach den Figuren 2 bis 11, bei der insbesondere Sensoreinheiten 66, 68, 98, 99, 140, 142 zum Steuern der Wertscheinzufuhr in das Ausgabefach 72 und zur Wertscheinausgabe schematisch dargestellt sind.

Zum Zuführen der Wertscheine 48 in das Ausgabefach 72 wird das Ablageelement 62 mit Hilfe der Antriebseinheit 94 in eine erste Zuführposition verfahren. Zum Verfahren des Ablageelements 62 in die erste Zuführposition ist die Steuereinheit 44 dazu ausgebildet, in einem ersten Schritt die Antriebseinheit 94 zum Bewegen des Ablageelements 62 in Abhängigkeit der von den Sensoreinheiten 98 und 99 ausgegebenen Sensorsignale anzusteuern. Dabei wird das Ablageelement 62 von einer Ausgangsposition, beispielsweise einer unteren, mit Hilfe der Sensoreinheit 140 erfassbaren Position, soweit nach oben in Richtung des Begrenzungselements 74 verfahren, bis das Ablageelement 62 die am Begrenzungselement 74 angelenkten Andruckelemente 96, 97 kontaktiert, die durch das Ablageelement 62 in die zweite Andruckposition gedrückt werden. Die zweite Andruckposition wird mit Hilfe der Sensoreinheiten 98 und 99 detektiert, die das Sensorsignal an die Steuereinheit 44 übermitteln.

Zu dem Zeitpunkt, in dem die zweite Andruckposition der Andruckelemente 96, 97 von den Sensoreinheiten 98, 99 detektiert wird, ist das Ablageelement 62 in einer Initialisierungsposition angeordnet. Ausgehend vom Empfang der Sensorsignale der Sensoreinheiten 98, 97 steuert die Steuereinheit die Antriebseinheit 94 derart an, dass das Ablageelement 62 um eine voreingestellte Wegstrecke von der Intialisierungsposition bis in eine erste Zuführposition zum Zuführen der Wertscheine 48 vom Begrenzungselement 74 weg bewegt wird. Die Wegstrecke wird erfindungsgemäß durch eine voreingestellte Anzahl der Schritte eines Schrittmotors der Antriebseinheit 94 vorgegeben.

Sobald sich das Ablageelement 62 in der Zuführposition befindet, können Wertscheine 48 in das Ausgabefach 72 transportiert und dort abgelegt werden. Vorzugsweise steuert die Steuereinheit 44 ab dem Zeitpunkt, ab dem der Sensor 42 einen zur Auszahlung zulässigen Wertschein 48 detektiert und das entsprechende Sensorsignal an die Steuereinheit 44 übermittelt, die Antriebseinheit 76 derart an, dass eine Zufuhr der Wertscheine 48 in das Ausgebfach 72 möglich ist.

Zur Überwachung des Ausgabefachs 72 ist eine weitere Sensoreinheit 142, vorzugsweise eine analoge Lichtschrankenanordnung, vorgesehen, die ermittelt, ob mindestens ein auszuzahlender Wertschein 48 in dem Ausgabefach 72 abgelegt ist oder ob das Ausgabefach 72 leer ist. Bei der Detektion eines Wertscheins 48 im Ausgabefach 72 wird ein entsprechendes Sensorsignal an die Steuereinheit 44 übermittelt.

Wenn der Wertscheinstapel der im Ausgabefach 72 abgelegten Wertscheine 48 eine vorbestimmte Höhe erreicht und sich das Ablageelement 62 in der ersten Zuführposition befindet, werden die Andruckelemente 96, 97 in die zweite Andruckposition ausgelenkt bzw. verschwenkt, wobei die Sensoreinheiten 98, 99 das entsprechende Sensorsignal an die Steuereinheit 44 übertragen.

Ausgehend von der Detektion des Sensorsignals der Sensoreinheiten 98 und 99 und der Detektion des Sensorsignals des Sensors 142 steuert die Steuereinheit 44 die Antriebseinheit 94 zum Bewegen des Ablageelements 62 derart an, dass das Ablageelement 62 von dem Begrenzungselement 74 weg von der ersten Zuführposition in eine zweite Zuführposition bewegt wird. Dieser Vorgang kann vorzugsweise zweimal wiederholt werden, das heißt, dass noch eine dritte Zuführposition des Ablageelements 62 vorgesehen sein kann. Bei alternativen Ausführungsformen können auch mehr oder weniger als drei Zuführpositionen vorgesehen sein.

Nachdem alle zur Auszahlung vorgesehenen Wertscheine 48 in das Ausgabefach 72 transportiert worden sind, wird das Ausgabefach 72 geöffnet, sodass eine Bedienperson auf die im Ausgabefach 72 zur Entnahme bereitgestellten Wertscheine 48 zugreifen kann. Zum Öffnen des Ausgabefachs 72 ist die Steuereinheit 44 dazu ausgebildet, in einem ersten Schritt die Antriebseinheit 94 derart anzusteuern, dass das Ablageelement 62 ausgehend von der Ausgangsposition in der sich das Ablageelement 62 befindet, in eine Mitnahmeposition bewegt wird. Die Ausgangsposition kann die erste Zuführposition, die zweite Zuführposition, die dritte Zuführposition oder auch die untere, mit Hilfe der Sensoreinheit 140 detektierbare Position des Ablageelements 62 sein.

Die Mitnahmeposition ist exemplarisch in den Figuren 7 und 8 gezeigt und bezeichnet eine Position des Ablageelements 62, in der das Begrenzungselement 74 durch das Ablageelement 62, bzw. durch die auf dem Ablageelement 62 abgelegten Wertscheine 48 in die zweite Begrenzungsposition angehoben wird und zwar derart, dass die Eingriffselemente 102, 103 des Begrenzungselements 74 beim Öffnen des Verschlusselements 60 jeweils mit einem Vorsprung 132 des Verschlusselements 60 in Eingriff kommen, sodass das Begrenzungselement 74 zusammen mit dem Eingriffselement 102 um seine Drehachse D2 verschwenkbar ist.

In einem zweiten Schritt steuert die Steuereinheit 44 die Antriebseinheit 144 zum Öffnen des Verschlusselements 60 an, wobei die die Eingriffselemente 102, 103 und das Begrenzungselement 74 im Zuge der Öffnungsbewegung des Verschlusselements 60 in Eingriff mit dem Verschlusselement 60 gelangen und gemeinsam mit dem Verschlusselement 60 bewegt werden, bis das Verschlusselement 60 die in den Figuren 4 und 5 dargestellte geöffnete Position einnimmt. Im Zuge dieser gemeinsamen Bewegung des Begrenzungselements 74 mit dem Verschlusselement 60 wird das Begrenzungselement 74 von der zweiten Begrenzungsposition in die dritte, geöffnete Begrenzungsposition bewegt und von dem Verschlusselement 60 in der geöffneten Position gehalten.

Die Steuereinheit 44 steuert die Antriebseinheit 144 zum Öffnen des Verschlusselements 60 vorzugsweise in Abhängigkeit des Sensorsignals der Sensoreinheiten 66 und 68 an. Die Sensoreinheit 66 ermittelt die geschlossene Position und die Sensoreinheit 68 die geöffnete Position des Verschlusselements 60. Die Steuereinheit 44 steuert die Antriebseinheit 144 insbesondere derart an, dass das Verschlusselement 60 zur Entnahme der Wertscheine 48 für einen vorbestimmten Zeitraum in der geöffneten Position verbleibt und nach dem voreingestellten Zeitraum in die geschlossene Position bewegt wird.

Wertscheine 48, die aus dem Ausgabefach 72 nicht entnommen worden sind, müssen als sogenannte Retracts in den Reject-und Retractbehälter 54 transportiert werden. Zu diesem Zweck ist die Steuereinheit 44 dazu ausgebildet, zu prüfen, ob die Sensoreinheit 142 nach Ablauf des voreingestellten Zeitintervalls das Sensorsignal weiter zur Steuereinheit 44 überträgt. Falls diese Bedingung erfüllt ist, steuert die Steuereinheit 44 die Antriebseinheit 94 derart an, dass das Ablageelement 62 von der Mitnahmeposition in eine Abzugsposition bewegt wird, in der die Wertscheine 48 mit Hilfe der Abzugsrollen 92, 92a aus dem Ausgabefach 72 entnehmbar sind. Ferner steuert die Steuereinheit 44 die Antriebseinheit 76 derart an, dass die Abzugsrollen 92, 92a zum Transport der Wertscheine 48 in den Reject-Retractbehälter 54 angetrieben werden.

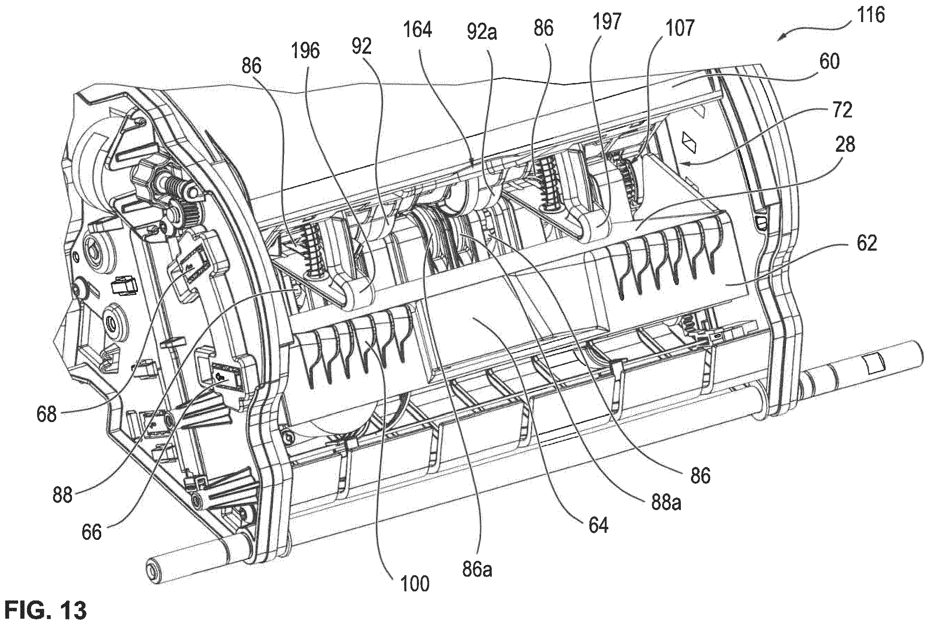

Figur 13 zeigt eine perspektivische Ansicht einer Wertscheinausgabeeinheit 116 gemäß einer zweiten Ausführungsform, die zum Einsatz in der Vorrichtung 10 nach Figur 1 alternativ zu der Wertscheinausgabeeinheit 16 nach den Figuren 2 bis 12 geeignet ist. Elemente mit gleichem Aufbau oder gleicher Funktion haben dieselben Bezugszeichen.

Die Darstellung nach Figur 13 ist eine perspektivische Detailansicht der Wertscheinausgabeeinheit 116 mit geöffnetem Verschlusselement 60. Die Wertscheinausgabeeinheit 116 hat denselben Aufbau und dieselbe Funktion wie die Wertscheinausgabeeinheit 16, jedoch sind die Andruckelemente 96, 97 durch die Andruckelemente 196 und 197 ersetzt worden. Der übrige Aufbau und die übrigen Funktionen sind identisch.

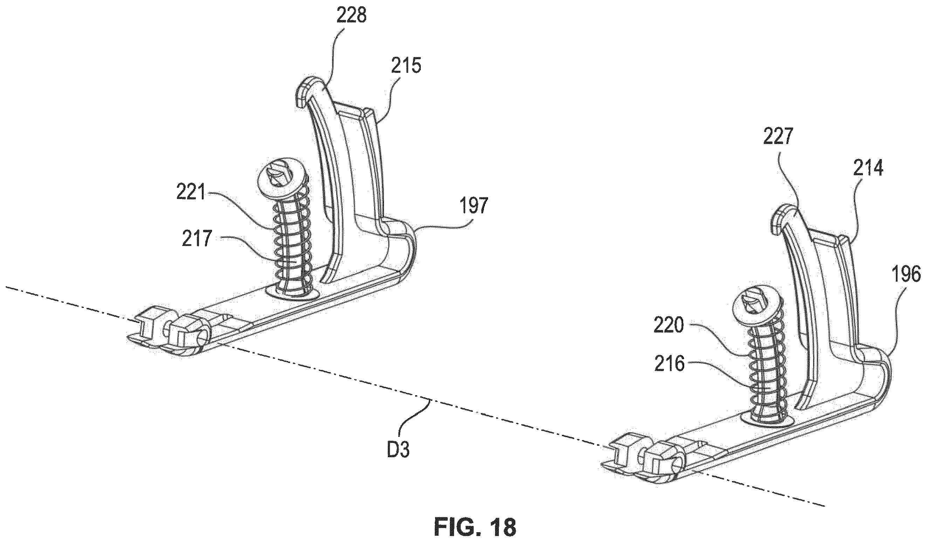

Im Unterschied zu den Andruckelementen 96, 97 sind die Andruckelemente 196, 197 nicht mit dem Begrenzungselement 76 verbunden sondern sind um eine feste Drehachse (D3 in Figur 18) schwenkbar angeordnet, so dass die Andruckelemente 196, 197 unabhängig vom Begrenzungselement 96 bewegbar sind. In Figur 13 ist ein Wertschein 28 im Ausgabefach 72 angeordnet, der durch die Andruckelemente 196, 197 auf dem Ablageelement 62 gehalten bzw. gegen das Ablageelement 62 gedrückt wird. Die Andruckelemente 196, 197 werden mit Hilfe von Federn 220, 221 in der in Figur 13 gezeigten Position des Ablageelements 62 gegen das Ablageelement 62 gedrückt. Zur Führung der Bewegung hat das Andruckelement 196 einen Führungsstab 216, der in eine Führungsöffnung 218 ragt und bei einer Bewegung des Andruckelements 196 entgegen der Federkraft und in Richtung der Federkraft geführt wird. Die Feder 220 ist als Spiralfeder ausgebildet und um den Führungsstab 216 herum angeordnet. Ein Ende der Feder 220 drückt gegen das Andruckelement 196. Das andere Ende der Feder drückt gegen ein festes Gehäuseteil des Ausgabefachs 62. In gleicher Weise ist beim Andruckelement 197 die Feder 221 um einen Führungsstab 217 herum angeordnet, wobei der Führungsstab 217 in eine Führungsöffnung 219 ragt und bei einer Bewegung des Andruckelements 197 in der Führungsöffnung 219 geführt wird.

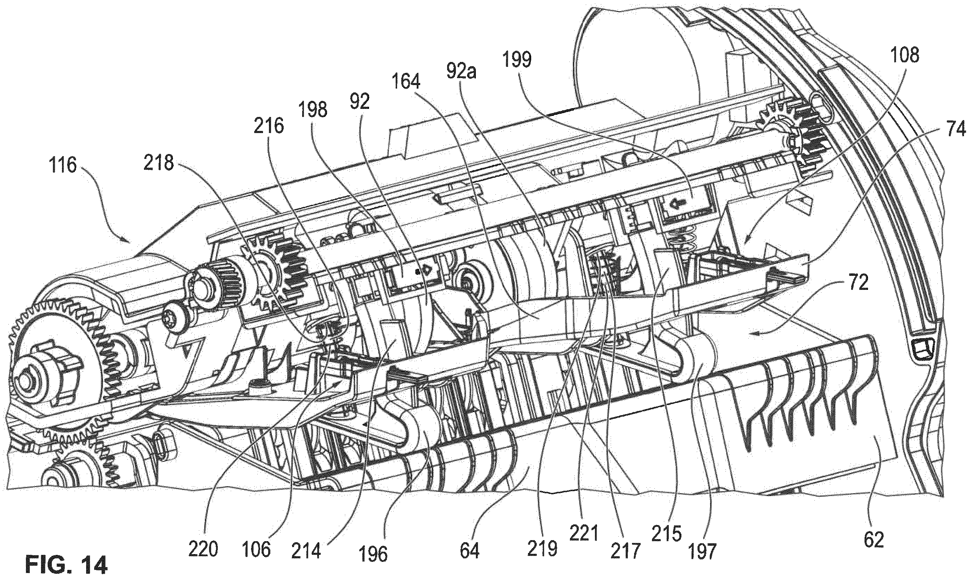

Figur 14 zeigt eine perspektivische Detailansicht eines Ausschnitts der Wertscheinausgabeeinheit 116 nach Figur 13. Zur vereinfachten Darstellung und besseren Übersichtlichkeit sind mehrere Elemente ausgeblendet worden. Gegenüber der Darstellung nach Figur 13 ist das Begrenzungselement 76 in einer unteren Position angeordnet. Ferner ist im Ausgabefach 72 kein Wertschein angeordnet. Die Andruckelemente 196, 197 sind trotz Veränderung der Lage des Begrenzungselements 76 gegenüber Figur 13 unverändert. Beim Stapeln mehrerer Wertscheine auf dem Ablageelement 62 werden die Andruckelemente 196, 197 angehoben. Die Andruckelemente 196, 197 haben jeweils ein Unterbrechungselement 214, 215. Beim Erreichen einer vorbestimmten Stapelhöhe der auf dem Ablageelement gestapelten Wertscheine 28 tauchen die Unterbrechungselemente 214, 215 in dem Erfassungsbereich einer als Lichtschrankenanordnung ausgebildeten Sensoreinheit 198, 199 ein und unterbrechen den Lichtstrahl zwischen dem nicht dargestellten Sender und nicht dargestellten Empfänger. Dadurch erhält die Steuereinheit 44 durch ein Sensorsignal der Sensoreinheiten 198, 199 eine Information, dass eine vorbestimmte Stapelhöhe der im Ausgabefach 72 befindlichen Wertscheine erreicht ist. Auch kann dieses Signal dazu benutzt werden, um die Höhe des Ablageelements 62 bei einer Initialisierung der Wertscheinausgabeeinheit 116 einzustellen, wie dies für die Wertscheinausgabeeinheit 16 in Verbindung mit den Andruckelementen 96, 97 und Sensoreinheiten 98, 99 beschrieben worden ist.

In gleicher Weise wie für die Andruckelemente 96 und 97 der Wertscheinausgabeeinheit 16 beschrieben, befinden sich die Andruckelemente 196, 197 in einer ersten Andruckposition und das Begrenzungselement 74 befindet sich in einer ersten Begrenzungsposition, bevor Wertscheine durch die Wertscheinausgabeeinheit 116 in dem Ausgabefach 72 abgelegt werden. Dabei kontaktiert das Andruckelement 196, 197 das Ablageelement 62. Wird dem Ausgabefach 72 ein Wertschein 28, 48 zugeführt, wird die Bewegung des Wertscheins durch die Andruckelemente 196, 197 abgebremst bzw. gestoppt. Zudem lenkt der zugeführte Wertschein 48 das Andruckelement 96 in Richtung der Sensoreinheit 198, 199 aus. Die Andruckelemente 196, 197 drücken den zugeführten Wertschein 48 schon beim Zuführen in das Ausgabefach 72 gegen das Ablageelement 62 und stellen dadurch eine geordnete Ablage der Wertscheine 28, 48 sicher.

Die zugeführten Wertscheine 28 48 bilden einen Wertscheinstapel, wobei der oberste Wertschein des Wertscheinstapels mit seiner Vorder- oder Rückseite die Andruckelemente 196, 197 kontaktiert. Je mehr Wertscheine im Ausgabefach 72 abgelegt werden, desto stärker ist die Auslenkung des Andruckelements 196, 197 in Richtung der Sensoreinheit 198, 199. Mit Hilfe der Sensoreinheiten 198, 199 wird eine zweite Andruckposition der Andruckelemente 196, 197 erfasst, die einer voreingestellten Auslenkung der Andruckelemente 196, 197 und somit einer voreingestellten Höhe des Wertscheinstapels entspricht. Hierzu unterbricht das jeweilige Unterbrechungselement 214, 215 die vom Sender der Sensoreinheiten 198, 199 ausgesandten Lichtstrahlen, wobei der jeweilige Empfänger die Unterbrechung detektiert, wodurch der Steuereinheit 44 ein Detektionssignal übermittelt wird.

Die Steuereinheit 44 steuert davon ausgehend die Antriebseinheit 94 an, die das Ablageelement 62 über eine Getriebeanordnung 95 um einen voreingestellten Abstand entgegen der Stapelrichtung R1 bewegt. Durch diese Bewegung werden die Andruckelemente 196, 197 aus der zweiten Andruckposition von den Sensoreinheiten 198, 199 weg bewegt, so dass die zweite Andruckposition der Andruckelemente 196, 197 nicht mehr durch die Sensoreinheiten 198, 199 erfasst wird. Darauf hin können weitere Wertscheine dem Ausgabefach 72 zugeführt werden, wobei der oben beschriebene Vorgang der Bewegung des Ablageelements 62 entgegen der Richtung R1 wiederholt wird, wenn die Andruckelemente 196, 197 von den abgelegten Wertscheinen ein weiteres mal bis in die zweite Andruckposition bewegt werden.

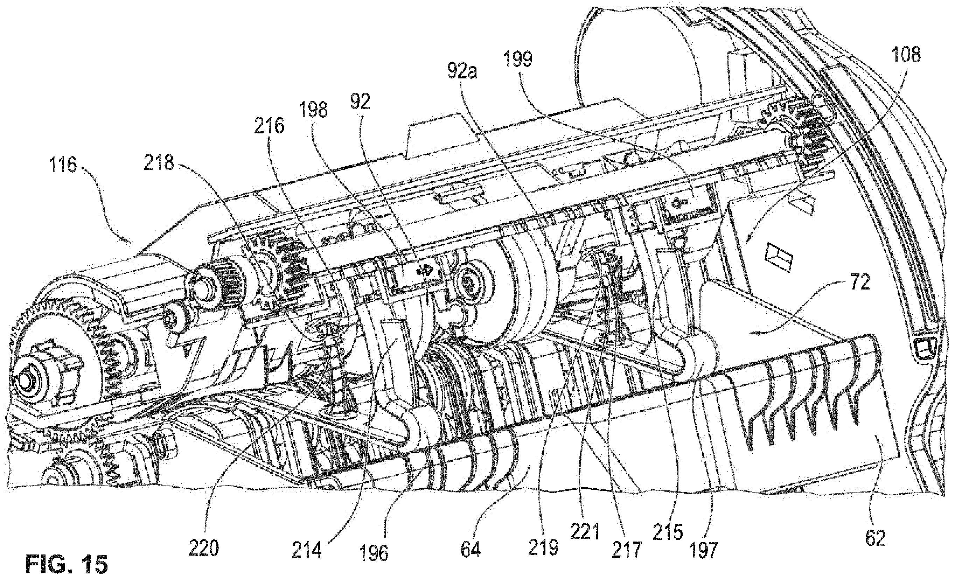

Figur 15 zeigt den Ausschnitt der Wertscheinausgabeeinheit 116 nach Figur 14, wobei das Begrenzungselement 74 die Wertscheinausgabeeinheit 116 ausgeblendet ist.

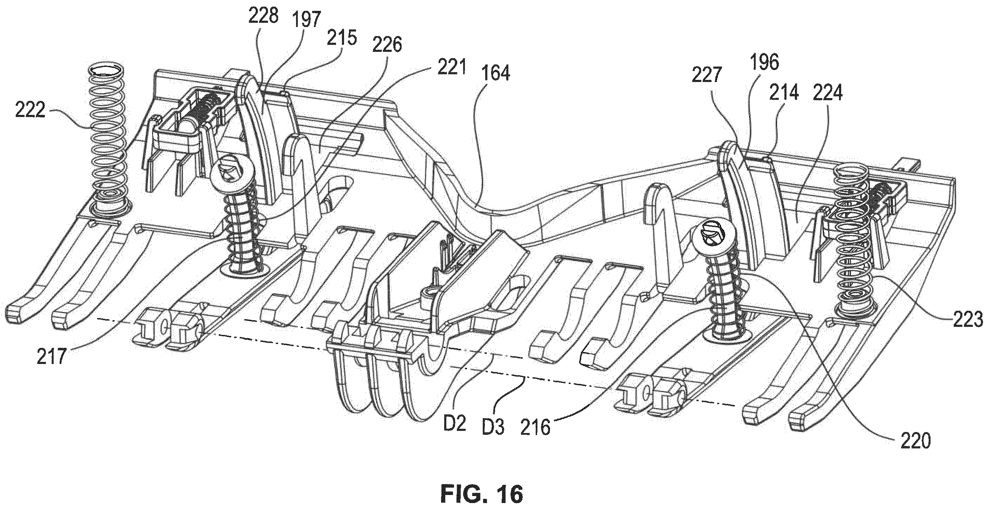

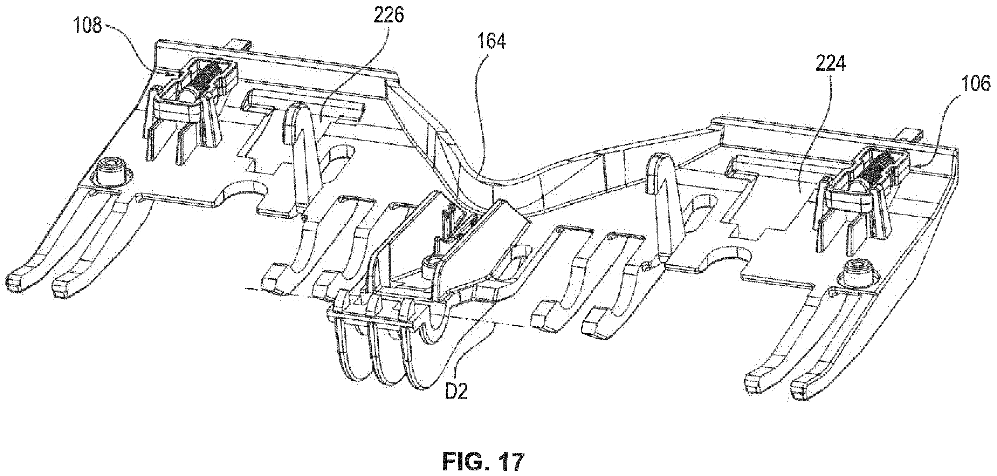

Figur 16 zeigt eine perspektivische Detailansicht des Ablageelements 74 und der Andruckelemente 196, 197 der Wertscheinausgabeeinheit 116 nach den Figuren 13 bis 15. Im vorliegenden Ausführungsbeispiel sind die Andruckelemente 196, 197 um eine in Figur 17 dargestellte Schwenkachse D3 und das Andruckelement 74 um eine in Figur 6 gezeigte Schwenkachse D2 schwenkbar angeordnet. Im vorliegenden Ausführungsbeispiel sind die Schwenkachsen D2 und D3 parallel und haben einen Abstand zueinander. Bei anderen Ausführungsbeispielen können die Drehachsen D2 und D3 auch auf einer Geraden liegen, so dass die Andruckelemente 196, 197 und das Begrenzungselement 74 um dieselbe Drehachse geschwenkt werden. Bei der Wertscheinausgabeeinheit 116 sind weitere Federelemente 222, 223 vorgesehen, die eine Kraft auf das Begrenzungselement 74 in Richtung des Ablageelements 62 ausüben, so dass das Begrenzungselement 74 sicher in einer unteren Position gehalten wird, wenn Wertscheine im Ablagefach 72 abgelegt werden oder das Verschlusselement 60 verschlossen ist. Wie in Figur 16 zu sehen ist, sind die Unterbrechungselemente 214, 215 durch Öffnungen 224, 226 im Begrenzungselement 74 geführt. Die Unterbrechungselemente 214, 215 sind jeweils mit Führungselementen 227, 228 der Andruckelemente 196, 197 in ihrer Bewegung relativ zum Begrenzungselement 74 geführt, wobei an dem dem Kontaktbereich mit dem Wertschein 28, 48 des jeweiligen Andruckelements 196, 197 gegenüberliegenden Ende des Führungselements 227, 228 ein Vorsprung ausgebildet ist, der als Anschlag dient. Durch den Vorsprung wird die Schwenkbewegung der Andruckelemente 196, 197 gegenüber dem Begrenzungselement 74 begrenzt, wenn der Vorsprung das Begrenzungselement 74 kontaktiert.

Figur 17 zeigt eine weitere perspektivische Detailansicht des Ablageelements 74 nach Figur 16 ohne Andruckelemente 196, 197.

Figur 18 zeigt eine perspektivische Detailansicht der Andruckelemente 196, 197 nach Figur 16.

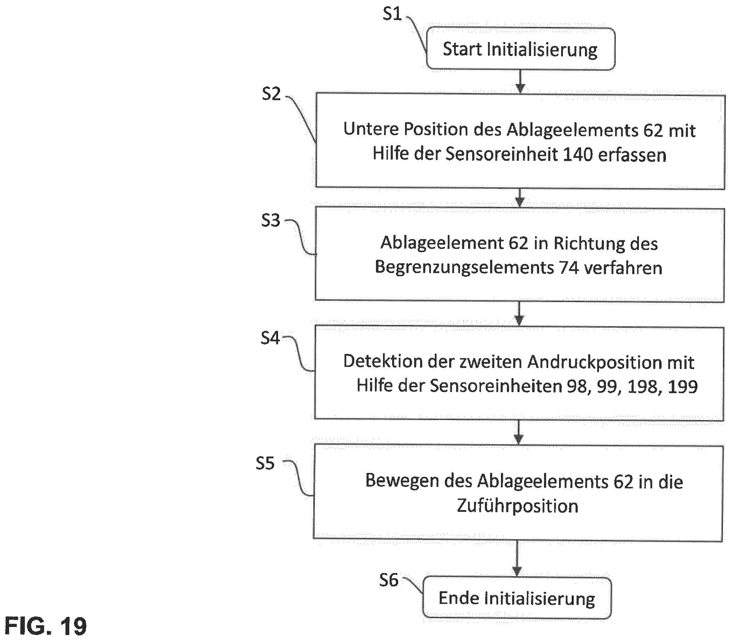

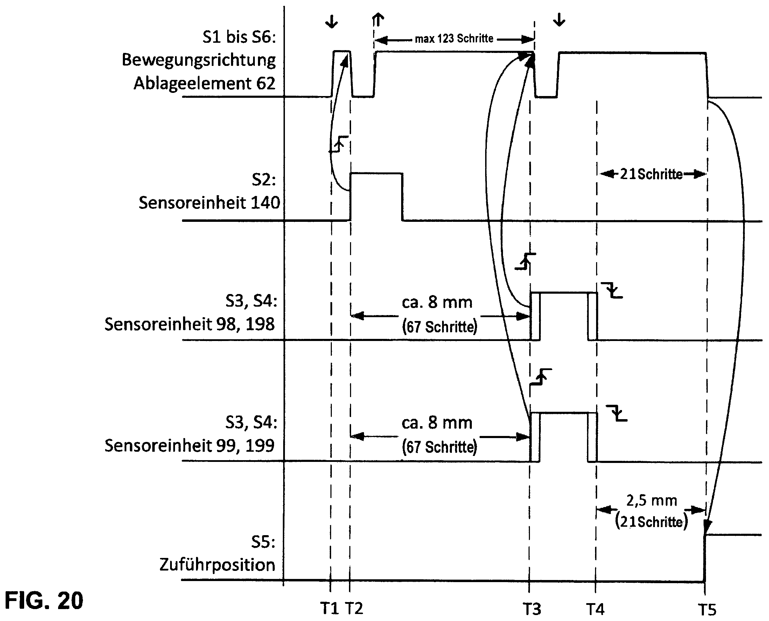

Figur 19 zeigt einen Ablaufplan eines Verfahrens zur Initialisierung der Wertscheinausgabeeinheit 16, 116 nach einer der Figuren 2 bis 18. Figur 20 zeigt ausgehend vom Ablaufplan nach Figur 19 ein Signaldiagramm zur Steuerung der Initialisierung einer Wertscheinausgabeeinheit 16, 116 nach einer der Figuren 2 bis 18.

Der Ablauf der Initialisierung wird mit dem Schritt S1 gestartet. Anschließend wird die untere Position des Ablageelements 62 mit Hilfe der Sensoreinheit 140 erfasst. Hierzu wird erforderlichenfalls das Ablageelement 62 mit Hilfe der Antriebseinheit 96 zum Bewegen des Ablageelements 62 bis in die untere Position bewegt. Anschließend wird im Schritt S3 das Ablageelement 62 mit Hilfe der Antriebseinheit 94 in Richtung des Begrenzungselements 74 verfahren. Im Schritt S4 wird dann geprüft, ob das Ablageelement 62 so weit in Richtung des Begrenzungselements 74 verfahren ist, bis das Ablageelement 62 die Andruckelemente 96, 97, 196, 197 soweit ausgelenkt hat, dass die Sensoreinheiten 98, 99, 198, 199 das jeweilige Unterbrechungselement 214, 215, 114, 115 detektieren. Wenn beide Sensoreinheiten 98, 99 bzw. 198, 199 eine zweite obere Position der Andruckeinheiten 96, 97, 196, 197 detektieren, wird die Bewegung des Ablageelements 62 in Richtung des Begrenzungselements 74 unterbrochen. Anschließend wird das Ablageelement 62 mit Hilfe der Antriebseinheit 94 im Schritt S5 wieder von dem Begrenzungselement 74 um eine vorbestimmte Strecke weg bewegt, so dass das Ablageelement 62 dann in der Zuführposition angeordnet ist, in der eine Ablage von Wertscheinen im Ausgabefach 72 möglich ist. Der Ablauf der Initialisierung ist dann im Schritt S6 beendet.

In dem in Figur 20 gezeigten Signaldiagramm ist in der obersten Zeile die Ansteuerung der Antriebseinheit 94 zum Bewegen des Ablageelements 62 gezeigt. In Zeile 2 ist der für Schritt S2 relevante Signalverlauf der Sensoreinheit 140 dargestellt, in Zeile 3 der für die Schritte S3 und S4 relevante Signalverlauf der Sensoreinheiten 98, 198, in Zeile 4 der für die Schritte S3 und S4 relevante Signalverlauf der Sensoreinheiten 99, 199 und in Zeile 5 der von der Steuereinheit 44 erzeugte Signalverlauf des Signals des Erreichens der Zuführposition.

Zum Zeitpunkt T1 wird die Antriebseinheit 94 zum Bewegen des Ablageelements 62 derart angesteuert, dass das Ablageelement 62 nach unten in die untere Position verfahren wird. Nachdem mit Hilfe der Sensoreinheit 140 zum Zeitpunkt T2 detektiert wurde (Schritt S2), dass die untere Position des Ablageelements 62 erreicht worden ist, wird das Ablageelement 62 mit Hilfe der Antriebseinheit in Richtung des Begrenzungselements 74 verfahren (Schritt S3), bis die Sensoreinheit 98, 99 bzw. 198, 199 jeweils eine zweite Position der Ablageelemente 96, 97 bzw. 196, 197 detektieren (Schritt S4) und die Bewegungen des Ablageelements 62 zum Zeitpunkt T3 gestoppt wird. Anschließend wird das Ablageelement 62 wieder vom Begrenzungselement 74 weg in Richtung der unteren Position bewegt. Nachdem die Sensoreinheiten 98, 99 bzw. 198, 199 zum Zeitpunkt T4 nicht mehr die zweite Position der Andruckelemente 96, 97, 196, 197 zum Zeitpunkt T4 detektieren, wird das Ablageelement 62 um eine voreingestellte Strecke weiter nach unten bewegt. Im vorliegenden Ausführungsbeispiel sind dies 21 Schritte des zum Antrieb des Ablageelements 62 dienenden Schrittmotors. Nach dem Ausführen der gesteuerten Bewegung zum Zeitpunkt T5 erzeugt die Steuereinheit 46 ein Signal, das angibt, dass das Ablageelement 62 die Zuführposition zum Zuführen von Wertscheinen in das Ablagefach 72 erreicht hat.

### Bezugszeichenliste

- 10: Vorrichtung
- 12: Kopfmodul
- 14: Tresormodul
- 16,116: Wertscheinausgabeeinheit
- 18: Übergabeöffnung
- 20 bis 26: Wertscheinkassette
- 28, 48, 50: Wertschein
- 30 bis 36: Vereinzelungseinheit
- 40: Transportpfad
- 42, 66, 68, 98, 99, 140 142, 144, 198, 199: Sensoreinheit
- 44: Steuereinheit
- 46: Weicheneinheit
- 52: Zwischenspeicher
- 54: Reject- und Retractbehälter
- 60: Verschlusselement
- 62: Ablageelement
- 64, 164: Aussparung
- 70: Transporteinheit
- 71: Umlenkrolle
- 72: Ausgabefach
- 73: Transportriemen
- 74: Begrenzungselement
- 76, 94, 144: Antriebseinheit
- 80, 82: Welle
- 92, 92a: Abzugsrolle
- 84, 84a, 86, 86a: Transportrolle
- 88, 88a, 88b: Flügelrad
- 90: Andruckrolle
- 93, 93a, 93b, 93c: Abzugsmittel
- 95: Getriebeanordnung
- 96, 97, 196, 197: Andruckelement
- 100: Rippenelement
- 102, 103: Eingriffselement
- 106, 108, 220, 221, 222, 223: Feder
- 114,115, 214, 215: Unterbrecherelement
- 110, 112: Sender
- 111, 113: Empfänger
- 122: Bodenelement
- 124: Abdeckelement
- 130: Spitze
- 132: Vorsprung
- 216, 217: Führungsstab
- 218, 219: Führungsöffnung
- 224, 226: Öffnung
- 227, 228: Führungselement
- D1, D2, D3: Drehachse
- R1, R2, T1: Richtung
- S1, S2: Schwenkradius

## Patentansprüche

1. *Vorrichtung zur Handhabung von Wertscheinen (28, 48, 50),*
*mit einem Ausgabefach (72) zur Entnahme von Wertscheinen (48), das ein das Ausgabefach (72)* auf *einer ersten Seite begrenzendes Ablageelement (62) und ein das Ausgabefach (72)* auf *einer zweiten Seite begrenzendes Begrenzungselement (74) umfasst,*
*mit einer Steuereinheit (44),*
wobei *die Vorrichtung mindestens eine erste Sensoreinheit (98, 99, 198, 199) zum Erfassen einer Initialisierungsposition des Ablageelements (62) umfasst,*
*dass die erste Sensoreinheit (98, 99, 198, 199) dazu ausgebildet ist, ein erstes Sensorsignal zu erzeugen und an die Steuereinheit (44) zu übermitteln,*
*dass die Steuereinheit (44) dazu ausgebildet ist, eine erste Antriebseinheit (94) zum Bewegen des Ablageelements (62) anzusteuern,*
*wobei die Steuereinheit (44) ausgehend von dem ersten Sensorsignal die erste Antriebseinheit (94) derart ansteuert, dass das Ablageelement (62)* um *eine vorbestimmte Strecke von dem Begrenzungselement (74) weg von der Initialisierungsposition in eine erste Zuführposition zum Zuführen der Wertscheine (48)* in das Ausgabefach *bewegt wird,*
**dadurch gekennzeichnet, dass**
*die* eine *vorbestimmte Strecke eine voreingestellte Wegstrecke ist, die durch eine voreingestellte Anzahl der Schritte eines Schrittmotors der Antriebseinheit (94) vorgegeben ist.*

2. *Vorrichtung nach Anspruch 1, **dadurch gekennzeichnet, dass** die erste Sensoreinheit (98, 99, 198, 199) mindestens ein Sensorelement (96, 97, 196, 197) umfasst, wobei die erste Sensoreinheit (98, 99, 198, 199) derart angeordnet ist, dass mit Hilfe der ersten Sensoreinheit (98, 99, 198, 199) eine erste Position des Sensorelements detektierbar ist, und dass die erste Sensoreinheit (98, 99, 198, 199) dazu ausgebildet ist, das erste Sensorsignal in Abhängigkeit von der detektieren ersten Position des Sensorelements (96, 97, 196, 197) auszugeben.*

3. *Vorrichtung nach Anspruch 2, **dadurch gekennzeichnet, dass** die erste Sensoreinheit (98, 99, 198, 199) die erste Position des Sensorelements detektiert, wenn sich das Ablageelement (62) in der Initialisierungsposition befindet, wobei das Sensorelement (96, 97, 196, 197) das Ablageelement (62) kontaktiert und durch den Kontakt mit dem Ablageelement (62) in Richtung des Begrenzungselements (74) bewegt wird.*

4. *Vorrichtung nach einem der Ansprüche 2 oder 3, **dadurch gekennzeichnet, dass** das Sensorelement (96, 97, 196, 197) ein Andruckelement (96, 97, 196, 197) zur geordneten Ablage der Wertscheine (48) ist, das schwenkbar mit dem Begrenzungselement (74) verbunden oder unabhängig vom Begrenzungselement (74) schwenkbar ist, wobei das Sensorelement (96, 97, 196, 197) zwischen der abgesenkten ersten Position und einer angehobenen zweiten Position bewegbar ist.*

5. *Vorrichtung nach einem der vorhergehenden Ansprüche, **dadurch gekennzeichnet, dass** die Vorrichtung mindestens eine zweite Sensoreinheit (140) zum Erfassen einer unteren Position des Ablageelements (62) umfasst, wobei die zweite Sensoreinheit (140) dazu ausgebildet ist, ein zweites Sensorsignal zu erzeugen und* an *die Steuereinheit (44) zu übermitteln, und*
*dass die Steuereinheit (44) dazu ausgebildet ist, die erste Antriebseinheit* (94) *zum Bewegen des Ablageelements (62) in Abhängigkeit des zweiten Sensorsignals anzusteuern.*

6. *Vorrichtung nach Anspruch 5, **dadurch gekennzeichnet, dass** die Steuereinheit (44) dazu ausgebildet ist, ausgehend von dem zweiten Sensorsignal die erste Antriebseinheit (94) derart anzusteuern, dass das Ablageelement (62)* um *eine vorbestimmte Strecke zum Begrenzungselement (74) hin in die Initialisierungsposition bewegt wird.*

7. *Vorrichtung nach einem der vorhergehenden Ansprüche, **dadurch gekennzeichnet, dass** die Vorrichtung ein Verschlusselement (60) umfasst, das das Ausgabefach (72) in einer geschlossenen Position an einer dritten Seite begrenzt und das in einer geöffneten Position Zugriff auf im Ausgabefach (72) bereitgestellte Wertscheine (48) ermöglicht, wobei das Verschlusselement (60) zum Öffnen des Ausgabefachs (72) von der geschlossenen Position in eine geöffnete Position und zum Schließen des Ausgabefachs von der geöffneten Position in die geschlossene Position bewegbar ist.*

8. *Vorrichtung nach Anspruch 7, **dadurch gekennzeichnet, dass** die Vorrichtung mindestens eine dritte Sensoreinheit (66) zum Erfassen der geschlossenen Position und*/*oder mindestens eine vierte Sensoreinheit (68) zum Erfassen der geöffneten Position des Verschlusselements (60) umfasst,*
*dass die dritte Sensoreinheit (66) dazu ausgebildet ist, ein drittes Sensorsignal zu erzeugen und an die Steuereinheit (44) zu übermitteln und*/*oder dass die vierte Sensoreinheit (68) dazu ausgebildet ist, ein viertes Sensorsignal zu erzeugen und* an *die Steuereinheit (44) zu übermitteln, und*
*dass die Steuereinheit (44) dazu ausgebildet ist, eine zweite Antriebseinheit (144) zum Antrieb des Verschlusselements (60) in Abhängigkeit des dritten Sensorsignals und*/*oder des vierten Sensorsignals anzusteuern.*

9. *Vorrichtung nach einem der vorhergehenden Ansprüche, **dadurch gekennzeichnet, dass** die Vorrichtung eine* fünfte *Sensoreinheit (142) umfasst, die insbesondere in dem Ausgabefach (72) angeordnet ist, und die dazu ausgebildet ist, ein fünftes Sensorsignal zu erzeugen und an die Steuereinheit (44) zu übermitteln, wenn mindestens ein Wertschein (48) in dem Ausgabefach (72) aufgenommen ist.*

10. *Vorrichtung nach einem der Ansprüche 2 bis 9, **dadurch gekennzeichnet, dass** mehrere in dem Ausgabefach (72) aufgenommene Wertscheine (48) einen Wertscheinstapel bilden, wobei die Wertscheine (48) des Wertscheinstapels derart übereinander angeordnet sind, dass der unterste Wertschein (48) mit seiner Vorderseite oder seiner Rückseite auf dem Ablageelement (62) liegt und dass der oberste Wertschein (48) mit seiner Vorderseite oder seiner Rückseite das Sensorelement (96, 97, 196, 197) kontaktiert, wobei die Richtung von dem untersten Wertschein (48) zum obersten Wertschein (48) eine Stapelrichtung (R1) des Wertscheinstapels ist, und*
*dass die erste Sensoreinheit (98, 99, 198, 199) die erste Sensorelementposition detektiert, wenn der Wertscheinstapel das Sensorelement (96, 97, 196, 197) kontaktiert, wobei das Sensorelement (96, 97, 196, 197) durch den Kontakt mit dem Wertscheinstapel in Richtung des Begrenzungselements (74) bewegt wird.*

11. *Vorrichtung nach Anspruch 10, **dadurch gekennzeichnet, dass** die Steuereinheit* (44) *ausgehend von dem Empfang des ersten Sensorsignals und des fünften Sensorsignals die erste Antriebseinheit (94) derart ansteuert, dass das Ablageelement (62)* um *eine vorbestimmte Strecke von dem Begrenzungselement (74) weg von der ersten Zuführposition in eine zweite Zuführposition oder von er zweiten Zuführposition in eine dritte Zuführposition bewegt wird, derart, dass weitere Wertscheine (48) in das Ausgabefach (72) zuführbar sind.*

12. *Vorrichtung nach einem der Ansprüche 7 bis 11, **dadurch gekennzeichnet, dass** die Steuereinheit (44) zum Öffnen des Ausgabefachs (72) dazu ausgebildet ist,*
*die erste Antriebseinheit (94) derart anzusteuern, dass das Ablageelement (62) ausgehend von der unteren Position oder von der ersten Zuführposition oder von der zweiten Zuführposition oder von der dritten Zuführposition* um *eine vorbestimmte Strecke in eine Mitnahmeposition bewegt wird, wobei das Begrenzungselement (74) bei der Bewegung des Ablageelements (62) von einer ersten Begrenzungsposition in eine zweite Begrenzungsposition bewegt wird, und*
*die zweite Antriebseinheit (144) in Abhängigkeit des dritten Sensorsignals zum Bewegen des Verschlusselements (60) von der geschlossenen in die geöffnete Position anzusteuern,*
*wobei ein Eingriffselement (102, 103) des Begrenzungselements (74) mit einem Eingriffsbereich des Verschlusselements (60) in Eingriff gelangt.*

13. *Vorrichtung nach einem der vorhergehenden Ansprüche, **dadurch gekennzeichnet, dass** die erste Sensoreinheit (98, 99) und*/*oder die zweite Sensoreinheit (140) und*/*oder die dritte Sensoreinheit (66) und*/*oder die vierte Sensoreinheit (68) und*/*oder die fünfte Sensoreinheit (142) mindestens ein Detektionselement umfasst, wobei das Detektionselement das Sensorsignal erzeugt und an die die Steuereinheit überträgt, und wobei das Detektionselement vorzugsweise eine Lichtschranke umfasst.*

14. *Vorrichtung nach einem der vorhergehenden Ansprüche, **dadurch gekennzeichnet, dass** die Vorrichtung mindestens eine dritte Antriebseinheit (76) zum Antrieb von Transportelementen zum Transport der Wertscheine (48) umfasst, wobei die Transportelemente zum Zuführen der Wertscheine (48) in das Ausgabefach (72) in eine erste Richtung antreibbar sind und wobei die Transportelemente in eine zweite, der ersten Richtung entgegengesetzte Richtung zum Transport der Wertscheine (48) aus dem Ausgabefach (72) heraus in Richtung einer Wertscheinhandhabungseinheit der Vorrichtung (10) antreibbar sind, und*
*dass die Vorrichtung eine sechste Sensoreinheit zur Detektion mindestens eines Merkmals der auszuzahlenden Wertscheine (48) umfasst, wobei die Steuereinheit (44) dazu ausgebildet ist, in Abhängigkeit des durch die sechste Sensoreinheit detektierten Merkmals zu unterscheiden, ob der Wertschein ein für die Auszahlung zulässiger Wertschein (48) ist, und*
*dass die Steuereinheit (44) die dritte Antriebseinheit (76) in die erste Richtung antreibt, wenn die sechste Sensoreinheit einen zur Auszahlung zulässigen Wertschein (48) detektiert.*

15. *Vorrichtung nach einem der Anspruch 12, **dadurch gekennzeichnet, dass** die Steuereinheit (44) dazu ausgebildet ist, die zweite Antriebseinheit (144) derart anzusteuern, dass das Verschlusselement (60) zur Entnahme der Wertscheine (48) für einen vorbestimmten Zeitraum in der geöffneten Position angeordnet ist und nach dem voreingestellten Zeitraum in die geschlossene Position bewegt wird und*
*dass die Steuereinheit (44) dazu ausgebildet ist, zu prüfen, ob die fünfte Sensoreinheit (142) nach Ablauf des voreingestellten Zeitraums das fünfte Sensorsignal weiter zur Steuereinheit (44) überträgt,*
*und dass die Steuereinheit (44), falls die Prüfung ergibt, dass das fünfte Sensorsignal weiter zur Steuereinheit übertragen wird, dazu ausgebildet ist,*
*die erste Antriebseinheit derart anzusteuern, dass das Ablageelement (62) von der Mitnahmeposition in eine Abzugsposition bewegt wird, und*
*die dritte Antriebseinheit (76) derart anzusteuern, dass die Transportelemente in die zweite Richtung angetrieben werden.*

## Claims

1. Device for handling bills (28, 48, 50),
having a dispensing compartment (72) for retrieving bills (48) which comprises a depositing element (62) that delimits the dispensing compartment (72) on a first side, and a delimiting element (74) that delimits the dispensing compartment (72) on a second side;
having a control unit (44),
wherein
the device comprises at least one first sensor unit (98, 99, 198, 199) for detecting an initializing position of the depositing element (62);
that the first sensor unit (98, 99, 198, 199) is configured to generate a first sensor signal and to transmit the latter to the control unit (44);
that the control unit (44) is configured to actuate a first drive unit (94) for moving the depositing element (62);
wherein the control unit (44), proceeding from the first sensor signal, actuates the first drive unit (94) in such a manner that the depositing element (62) is moved into the dispensing compartment by a predetermined distance away from the delimiting element (74), from the initializing position to a first feeding position for feeding the bills (48),
**characterized in that** the one predetermined distance is a preset distance which is predefined by a preset number of steps of a stepper motor of the drive unit (94).

2. Device according to Claim 1, **characterized in that** the first sensor unit (98, 99, 198, 199) comprises at least one sensor element (96, 97, 196, 197), wherein the first sensor unit (98, 99, 198, 199) is disposed in such a manner that a first position of the sensor element is able to be detected with the aid of the first sensor unit (98, 99, 198, 199), and **in that** the first sensor unit (98, 99, 198, 199) is configured to emit the first sensor signal as a function of the detected first position of the sensor element (96, 97, 196, 197).

3. Device according to Claim 2, **characterized in that** the first sensor unit (98, 99, 198, 199) detects the first position of the sensor element when the depositing element (62) is situated in the initializing position, wherein the sensor element (96, 97, 196, 197) contacts the depositing element (62) and as a result of contacting the depositing element (62) is moved in the direction of the delimiting element (74).

4. Device according to one of Claims 2 or 3, **characterized in that** the sensor element (96, 97, 196, 197) is a contact pressure element (96, 97, 196, 197) for the orderly depositing of the bills (48) which is pivotably connected to the delimiting element (74) or is pivotable independently of the delimiting element (74), wherein the sensor element (96, 97, 196, 197) is movable between the lowered first position and a raised second position.

5. Device according to one of the preceding claims, **characterized in that** the device comprises at least one second sensor unit (140) for detecting a lower position of the depositing element (62), wherein the second sensor unit (140) is configured to generate a second sensor signal and to transmit the latter to the control unit (44); and
**in that** the control unit (44) is configured to actuate the first drive unit (94) for moving the depositing element (62) as a function of the second sensor signal.

6. Device according to Claim 5, **characterized in that** the control unit (44), proceeding from the second sensor signal, is configured to actuate the first drive unit (94) in such a manner that the depositing element (62) is moved by a predetermined distance toward the delimiting element (74), to the initializing position.

7. Device according to one of the preceding claims, **characterized in that** the device comprises a closure element (60) which in a closed position delimits the dispensing compartment (72) on a third side, and which in an opened position enables access to bills (48) provided in the dispensing compartment (72), wherein the closure element (60) for opening the dispensing compartment (72) is movable from the closed position to an opened position, and for closing the dispensing compartment is movable from the opened position to the closed position.

8. Device according to Claim 7, **characterized in that** the device comprises at least one third sensor unit (66) for detecting the closed position and/or at least one fourth sensor unit (68) for detecting the opened position of the closure element (60);
**in that** the third sensor unit (66) is configured to generate a third sensor signal and to transmit the latter to the control unit (44), and/or **in that** the fourth sensor unit (68) is configured to generate a fourth sensor signal and to transmit the latter to the control unit (44); and
**in that** the control unit (44) is configured to actuate a second drive unit (144) for driving the closure element (60) as a function of the third sensor signal and/or of the fourth sensor signal.

9. Device according to one of the preceding claims, **characterized in that** the device comprises a fifth sensor unit (142) which is in particular disposed in the dispensing compartment (72) and which is configured to generate a fifth sensor signal and to transmit the latter to the control unit (44) when at least one bill (48) is received in the dispensing compartment (72).

10. Device according to one of Claims 2 to 9, **characterized in that** a plurality of bills (48) received in the dispensing compartment (72) form a bill stack, wherein the bills (48) of the bill stack are disposed on top of one another in such a manner that the lowermost bill (48) by way of the front side thereof or the rear side thereof bears on the depositing element (62) and that the uppermost bill (48) by way of the front side thereof or the rear side thereof contacts the sensor element (96, 97, 196, 197), wherein the direction from the lowermost bill (48) to the uppermost bill (48) is a stacking direction (R1) of the bill stack; and
**in that** the first sensor unit (98, 99, 198, 199) detects the first sensor element position when the bill stack contacts the sensor element (96, 97, 196, 197), wherein the sensor element (96, 97, 196, 197) as a result of contacting the bill stack is moved in the direction of the delimiting element (74).

11. Device according to Claim 10, **characterized in that** the control unit (44), proceeding from the reception of the first sensor signal and of the fifth sensor signal, actuates the first drive unit (94) in such a manner that the depositing element (62) is moved by a predetermined distance away from the delimiting element (74), from the first feeding position to a second feeding position, or from the second feeding position to a third feeding position, in such a manner that further bills (48) are able to be fed into the dispensing compartment (72).

12. Device according to one of Claims 7 to 11, **characterized in that** the control unit (44) for opening the dispensing compartment (72) is configured to
actuate the first drive unit (94) in such a manner that the depositing element (62), proceeding from the lower position or from the first feeding position or from the second feeding position or from the third feeding position, is moved by a predetermined distance to an entrainment position, wherein the delimiting element (74) in the movement of the depositing element (62) is moved from a first delimiting position to a second delimiting position; and
to actuate the second drive unit (144) as a function of the third sensor signal for moving the closure element (60) from the closed to the opened position;
wherein an engagement element (102, 103) of the delimiting element (74) comes to engage with an engagement region of the closure element (60).

13. Device according to one of the preceding claims, **characterized in that** the first sensor unit (98, 99) and/or the second sensor unit (140) and/or the third sensor unit (66) and/or the fourth sensor unit (68) and/or the fifth sensor unit (142) comprise(s) at least one detection element, wherein the detection element generates the sensor signal and transmits the latter to the control unit, and wherein the detection element preferably comprises a light barrier.

14. Device according to one of the preceding claims, **characterized in that** the device comprises at least one third drive unit (76) for driving transport elements for transporting the bills (48), wherein the transport elements for feeding the bills (48) into the dispensing compartment (72) are drivable in a first direction, and wherein the transport elements for transporting the bills (48) out of the dispensing compartment (72) in a direction of a bill handling unit of the device (10) are drivable in a second direction, counter to the first direction; and
**in that** the device comprises a sixth sensor unit for detecting at least one feature of the bills (48) to be disbursed, wherein the control unit (44) as a function of the feature detected by the sixth sensor unit is configured to distinguish whether the bill is a bill (48) cleared for disbursement; and
**in that** the control unit (44) drives the third drive unit (76) in the first direction when the sixth sensor unit detects a bill (48) cleared for disbursement.

15. Device according to one of Claim 12, **characterized in that** the control unit (44) is configured to actuate the second drive unit (144) in such a manner that the closure element (60) for retrieving the bills (48) is disposed in the opened position for a predetermined period, and after the pre-set period is moved to the closed position; and
**in that** the control unit (44) is configured to check whether the fifth sensor unit (142), after the pre-set period has elapsed, relays the fifth sensor signal to the control unit (44);
and **in that**, should the check result in the fifth sensor signal being relayed to the control unit, the control unit (44) is configured
to actuate the first drive unit in such a manner that the depositing element (62) is moved from the entrainment position to a discharge position; and
to actuate the third drive unit (76) in such a manner that the transport elements are driven in the second direction.

## Revendications

1. Dispositif de manipulation de documents de valeur (28, 48, 50),
avec un compartiment de distribution (72) pour prélever des documents de banque (48), qui comprend un élément de dépôt (62) délimitant le compartiment de distribution (72) sur un premier côté et un élément de délimitation (74) délimitant le compartiment de distribution (72) sur un deuxième côté,
avec une unité de commande (44),
wobei
le dispositif comprenant au moins une première unité de capteur (98, 99, 198, 199) pour détecter une position d'initialisation de l'élément de dépôt (62),
la première unité de capteur (98, 99, 198, 199) étant réalisée pour générer un premier signal de capteur et le transmettre à l'unité de commande (44),
l'unité de commande (44) étant réalisée pour commander une première unité d'entraînement (94) pour déplacer l'élément de dépôt (62),
l'unité de commande (44), sur la base du premier signal de capteur, commandant la première unité d'entraînement (94) de telle sorte que l'élément de dépôt (62) est déplacé d'une distance prédéterminée par rapport à l'élément de délimitation (74) depuis la position d'initialisation jusqu'à une première position d'amenée pour amener les documents de valeur (48) dans le compartiment de distribution,
**caractérisé en ce que** la distance prédéterminée est une distance de parcours préétablie indiquée par un nombre préétabli de pas d'un moteur pas à pas de l'unité d'entraînement (94).

2. Dispositif selon la revendication 1, **caractérisé en ce que** la première unité de capteur (98, 99, 198, 199) comprend au moins un élément de capteur (96, 97, 196, 197), la première unité de capteur (98, 99, 198, 199) étant agencée de telle sorte qu'à l'aide de la première unité de capteur (98, 99, 198, 199), une première position de l'élément de capteur peut être détectée, et **en ce que** la première unité de capteur (98, 99, 198, 199) est réalisée pour émettre le premier signal de capteur en fonction de la première position détectée de l'élément de capteur (96, 97, 196, 197).

3. Dispositif selon la revendication 2, **caractérisé en ce que** la première unité de capteur (98, 99, 198, 199) détecte la première position de l'élément de capteur lorsque l'élément de dépôt (62) se trouve dans la position d'initialisation, l'élément de capteur (96, 97, 196, 197) étant en contact avec l'élément de dépôt (62) et étant déplacé en direction de l'élément de délimitation (74) par le contact avec l'élément de dépôt (62).

4. Dispositif selon l'une quelconque des revendications 2 ou 3, **caractérisé en ce que** l'élément de capteur (96, 97, 196, 197) est un élément de pression (96, 97, 196, 197) pour le dépôt ordonné des documents de valeur (48), qui est relié de manière pivotante à l'élément de délimitation (74) ou qui peut pivoter indépendamment de l'élément de délimitation (74), l'élément de capteur (96, 97, 196, 197) pouvant être déplacé entre la première position abaissée et une deuxième position relevée.

5. Dispositif selon l'une quelconque des revendications précédentes, **caractérisé en ce que** le dispositif comprend au moins une deuxième unité de capteur (140) pour détecter une position inférieure de l'élément de dépôt (62), la deuxième unité de capteur (140) étant réalisée pour générer un deuxième signal de capteur et le transmettre à l'unité de commande (44), et **en ce que** l'unité de commande (44) est réalisée pour commander la première unité d'entraînement (94) pour déplacer l'élément de dépôt (62) en fonction du deuxième signal de capteur.

6. Dispositif selon la revendication 5, **caractérisé en ce que** l'unité de commande (44) est réalisée pour commander, à partir du deuxième signal de capteur, la première unité d'entraînement (94) de telle sorte que l'élément de dépôt (62) est déplacé d'une distance prédéterminée vers l'élément de délimitation (74) dans la position d'initialisation.

7. Dispositif selon l'une quelconque des revendications précédentes, **caractérisé en ce que** le dispositif comprend un élément de fermeture (60) qui, dans une position fermée, délimite le compartiment de distribution (72) sur un troisième côté et qui, dans une position ouverte, permet d'accéder à des documents de valeur (48) fournis dans le compartiment de distribution (72), l'élément de fermeture (60) pouvant être déplacé pour ouvrir le compartiment de distribution (72) de la position fermée à une position ouverte et pour fermer le compartiment de distribution de la position ouverte à la position fermée.

8. Dispositif selon la revendication 7, **caractérisé en ce que** le dispositif comprend au moins une troisième unité de capteur (66) pour détecter la position fermée et/ou au moins une quatrième unité de capteur (68) pour détecter la position ouverte de l'élément de fermeture (60),
**en ce que** la troisième unité de capteur (66) est réalisée pour générer un troisième signal de capteur et le transmettre à l'unité de commande (44) et/ou **en ce que** la quatrième unité de capteur (68) est réalisée pour générer un quatrième signal de capteur et le transmettre à l'unité de commande (44), et
**en ce que** l'unité de commande (44) est réalisée pour commander une deuxième unité d'entraînement (144) pour entraîner l'élément de fermeture (60) en fonction du troisième signal de capteur et/ou du quatrième signal de capteur.

9. Dispositif selon l'une quelconque des revendications précédentes, **caractérisé en ce que** le dispositif comprend une cinquième unité de capteur (142), agencée notamment dans le compartiment de distribution (72), et réalisée pour générer un cinquième signal de capteur et le transmettre à l'unité de commande (44) lorsqu'au moins un document de valeur (48) est reçu dans le compartiment de distribution (72).

10. Dispositif selon l'une quelconque des revendications 2 à 9, **caractérisé en ce que** plusieurs documents de valeur (48) reçus dans le compartiment de distribution (72) forment une pile de documents de valeur, les documents de valeur (48) de la pile de documents de valeur étant superposés de telle sorte que le document de valeur le plus bas (48) repose avec son côté avant ou son côté arrière sur l'élément de dépôt (62) et que le document de valeur le plus haut (48) entre en contact avec l'élément de capteur (96, 97, 196, 197) avec son côté avant ou son côté arrière, la direction allant du document de valeur le plus bas (48) au document de valeur le plus haut (48) étant une direction d'empilage (R1) de la pile de documents de valeur, et
**en ce que** la première unité de capteur (98, 99, 198, 199) détecte la première position d'élément de capteur lorsque la pile de documents de valeur entre en contact avec l'élément de capteur (96, 97, 196, 197), l'élément de capteur (96, 97, 196, 197) étant déplacé en direction de l'élément de délimitation (74) par le contact avec la pile de documents de valeur.

11. Dispositif selon la revendication 10, **caractérisé en ce que** l'unité de commande (44), sur la base de la réception du premier signal de capteur et du cinquième signal de capteur, commande la première unité d'entraînement (94) de telle sorte que l'élément de dépôt (62) est déplacé d'une distance prédéterminée par rapport à l'élément de délimitation (74) depuis la première position d'amenée jusqu'à une deuxième position d'amenée ou depuis la deuxième position d'amenée jusqu'à une troisième position d'amenée, de telle sorte que d'autres documents de valeur (48) peuvent être amenés dans le compartiment de distribution (72).

12. Dispositif selon l'une quelconque des revendications 7 à 11, **caractérisé en ce que** l'unité de commande (44) pour ouvrir le compartiment de distribution (72) est réalisée
pour commander la première unité d'entraînement (94) de telle sorte que l'élément de dépôt (62) est déplacé d'une distance prédéterminée depuis la position inférieure ou depuis la première position d'amenée ou depuis la deuxième position d'amenée ou depuis la troisième position d'amenée jusqu'à une position d'entraînement, l'élément de délimitation (74) étant déplacé d'une première position de délimitation à une deuxième position de délimitation lors du déplacement de l'élément de dépôt (62), et
pour commander la deuxième unité d'entraînement (144) en fonction du troisième signal de capteur pour déplacer l'élément de fermeture (60) de la position fermée à la position ouverte,
un élément de mise en prise (102, 103) de l'élément de délimitation (74) venant en prise avec une zone de mise en prise de l'élément de fermeture (60).

13. Dispositif selon l'une quelconque des revendications précédentes, **caractérisé en ce que** la première unité de capteur (98, 99) et/ou la deuxième unité de capteur (140) et/ou la troisième unité de capteur (66) et/ou la quatrième unité de capteur (68) et/ou la cinquième unité de capteur (142) comprend au moins un élément de capteur, l'élément de capteur générant le signal de capteur et le transmettant à l'unité de commande, et l'élément de capteur comprenant de préférence une barrière photoélectrique.

14. Dispositif selon l'une quelconque des revendications précédentes, **caractérisé en ce que** le dispositif comprend au moins une troisième unité d'entraînement (76) pour entraîner des éléments de transport pour transporter les documents de valeur (48), les éléments de transport pouvant être entraînés dans une première direction pour amener les documents de valeur (48) dans le compartiment de distribution (72) et les éléments de transport pouvant être entraînés dans une deuxième direction opposée à la première direction pour transporter les documents de valeur (48) hors du compartiment de distribution (72) en direction d'une unité de manipulation de documents de valeur du dispositif (10), et
**en ce que** le dispositif comprend une sixième unité de capteur pour détecter au moins une caractéristique des documents de valeur (48) à payer, l'unité de commande (44) étant réalisée pour distinguer, en fonction de la caractéristique détectée par la sixième unité de capteur, si le document de valeur est un document de valeur (48) admissible pour le paiement, et
**en ce que** l'unité de commande (44) entraîne la troisième unité d'entraînement (76) dans la première direction lorsque la sixième unité de capteur détecte un document de valeur (48) admissible pour le paiement.

15. Dispositif selon l'une quelconque de la revendication 12, **caractérisé en ce que** l'unité de commande (44) est réalisée pour commander la deuxième unité d'entraînement (144) de telle sorte que l'élément de fermeture (60) est agencé dans la position ouverte pour le prélèvement des documents de valeur (48) pendant une période de temps prédéterminée et est déplacé dans la position fermée après la période de temps préétablie, et
**en ce que** l'unité de commande (44) est réalisée pour vérifier si la cinquième unité de capteur (142) continue à transmettre le cinquième signal de capteur à l'unité de commande (44) après l'expiration de la période de temps préétablie,
et **en ce que**, si la vérification révèle que le cinquième signal de capteur est en outre transmis à l'unité de commande, l'unité de commande (44) est réalisée
pour commander la première unité d'entraînement de telle sorte que l'élément de dépôt (62) est déplacé de la position d'entraînement à une position de retrait, et
pour commander la troisième unité d'entraînement (76) de telle sorte que les éléments de transport sont entraînés dans la deuxième direction.
